# EUROPEAN PATENT SPECIFICATION

(11) **EP 3 876 914 B1**
(45) Date of publication and mention of the grant of the patent: **02.12.2026**
(21) Application number: 19836868.0
(22) Date of filing: 08.11.2019
(51) Int. Cl.: A61K 9/51, C12N 15/11, A61P 27/02

(54) **MESSENGER RNA THERAPY FOR USE IN A TREATMENT OF OCULAR DISEASES**
MESSENGER-RNA-THERAPIE ZUR VERWENDUNG IN DER BEHANDLUNG VON AUGENERKRANKUNGEN
THÉRAPIE PAR L'ARN MESSAGER POUR L'UTILISATION DANS LE TRAITEMENT DES MALADIES OCULAIRES

(30) Priority: 09.11.2018 US 201862758105 P
(43) Date of publication of application: 15.09.2021
(73) Proprietor: Translate Bio, Inc., Waltham, MA 02451 (US)
(72) Inventor: ANDROSAVICH, John R., Lexington, MA 02421 (US); DeROSA, Frank, Lexington, MA 02421 (US); KARVE, Shrirang, Lexington, MA 02421 (US)
(74) Representative: Carpmaels & Ransford LLP
(86) International application number: PCT/US2019/060546
(87) International publication number: WO 2020/097511

(56) References cited:
- WO-A1-2018/165257
- US-A1- 2013 259 923
- US-A1- 2017 143 848

## Description

### BACKGROUND

Messenger RNA therapy (MRT) is promising new approach to treat a variety of diseases. MRT involves administration of messenger RNA (mRNA) to a patient in need of the therapy. The administered mRNA produces a protein or peptide encoded by the mRNA within the patient's body. Several hurdles exist in implementing an effective treatment strategy for ocular diseases and disorders, mainly due to the unique anatomy and physiology of the eye. The combination of static barriers such as different layers and regions of the eye, and dynamic barriers such as blood flow, lymphatic clearance and tear dilution pose a significant challenge for drug delivery.

US 2017/143848 A1 (Calias et al., 2017) relates to mRNA therapy for the treatment of ocular diseases. US 2013/259923 A1 (Bancel et al., 2013) relates to modified polynucleotides for the production of secreted proteins. WO 2018/165257 A1 (Translate Bio. Inc., 2018) relates to polyanionic delivery of nucleic acids.

### SUMMARY OF THE INVENTION

The present invention is defined by the claims. The invention provides a composition comprising an effective amount of mRNA encoding a protein for use in the treatment of an ocular disease or disorder in a subject in need thereof, wherein the treatment comprises administering the composition to an eye of a human subject via intravitreal administration or suprachoroidal administration, wherein the effective amount of mRNA administered to the human subject ranges from 5 µg to 100 µg mRNA, wherein the mRNA is encapsulated in a lipid nanoparticle, and the lipid nanoparticle comprises one or more cationic lipids, one or more non-cationic lipids and a PEG-modified lipid, wherein administering the composition results in expression of the protein or the peptide encoded by the mRNA in one or more cells located in the outer nuclear layer (ONL), the inner segment photoreceptors (IS), the outer segment photoreceptors (OS), the retinal pigmented epithelium layer (RPE) of the retinal tissue, the choroid, and/or the sclera of the eye, wherein the subject is suffering from a disease or disorder affecting the posterior retinal layers or a tissue of the posterior eye, and wherein the disease or disorder is selected from age-related macular degeneration (AMD), cytomegalovirus (CMV) retinitis, Leber's congenital amaurosis, Stargardt disease, Usher disease, chorioretinitis, retinal detachment, uveitis, uvetic macular edema, cyclitis, choroiditis, diffuse uveitis and scleritis.

References herein to methods of treatment using compositions should be interpreted as references to said compositions for use in said methods of treatment.

The present invention provides, among other things, effective methods and compositions for the treatment of ocular diseases, disorders or conditions based on messenger RNA (mRNA) therapy. The present invention is, in part, based on unexpected observation that mRNA may be effectively delivered to the retina, choroid and/or sclera of the eye despite the uniquely challenging astatic and dynamic barriers due to complicated eye anatomy and physiology. Using lipid encapsulated mRNA, expansive retinal delivery can be achieved. Following administration of lipid-encapsulated mRNA formulations as described herein, are capable of reaching deep into the retinal tissue. Surprisingly, using the method and compositions described herein, very low quantities of mRNA can be effectively delivered to the deep tissues of the eye. Efficient delivery and expression of mRNA-encoded protein is achieved by administering low doses of mRNA as disclosed in the instant application. This implies that the method and compositions disclosed herein provide a therapeutic advantage of the mRNA over a range of doses. Therefore, the present invention provides an effective solution for this difficult and long-standing problem of ocular drug delivery.

Thus, in one aspect, the invention provides a method for ocular delivery of messenger RNA (mRNA), comprising administering to an eye of a subject in need thereof, a composition comprising: an effective amount of an mRNA encoding a protein or a peptide, wherein the mRNA is encapsulated in a lipid nanoparticle, and wherein administering the composition results in expression of the protein or the peptide encoded by the mRNA in one or more cells located in the outer nuclear layer (ONL), the inner segment photoreceptors (IS), the outer segment photoreceptors (OS), the retinal pigmented epithelium layer (RPE) of the retinal tissue, the choroid, and/or the sclera of the eye.

As defined in the claims, the mRNA is administered to the eye of the subject via intravitreal or suprachoroidal administration.

In some embodiments, administering the composition results in expression of the protein encoded by the mRNA in the retinal tissue. In some embodiments, administering the composition results in expression of the protein encoded by the mRNA in the choroid.

In some embodiments, administering the composition results in expression of the protein encoded by the mRNA in the sclera.

As defined in the claims, the subject to which the effective amount of mRNA is administered is human. The effective amount of mRNA administered to the human subject ranges from 5 µg to 100 µg mRNA. In some embodiments, the effective amount of mRNA administered to the human subject ranges from 10 µg to 80 µg mRNA. For example, the effective amount of mRNA may range from 30 µg to 60 µg mRNA. The effective amount of mRNA may be administered to the human subject by intravitreal administration (typically as a single injection). The effective amount of mRNA may be administered at a volume ranging from 30 µl about to about 100 µl.

As defined in the claims, the subject in need of treatment with the compositions of the invention is suffering from a disease or disorder affecting the posterior retinal layers or a tissue of the posterior eye. Diseases or disorders affecting the posterior retinal layers or a tissue of the posterior eye include age-related macular degeneration (AMD), cytomegalovirus (CMV) retinitis, Leber's congenital amaurosis, Stargardt disease, Usher disease, chorioretinitis, retinal detachment, uveitis, uvetic macular edema, cyclitis, choroiditis, diffuse uveitis and scleritis.

As defined in the claims, the subject is human. As defined in the claims, the effective amount of mRNA administered to the human subject ranges from 5 µg to 100 µg mRNA. In some embodiments, the effective amount of mRNA administered to the human subject ranges from 10 µg to 80 µg mRNA. In some embodiments, the effective amount of mRNA administered to the human subject ranges from 30 µg to 60 µg mRNA. In some embodiments, the composition is administered to the human subject by intravitreal injection. In some embodiments, the composition is administered to the human subject at a volume ranging from 30 µl about to about 100 µl.

As defined in the claims, the subject is suffering from a disease or disorder affecting the posterior retinal layers or a tissue of the posterior eye. As defined in the claims, the disease or disorder affecting the posterior retinal layers or the tissue of the posterior eye is selected from age-related macular degeneration (AMD), cytomegalovirus (CMV) retinitis, Leber's congenital amaurosis, Stargardt disease, Usher disease, chorioretinitis, retinal detachment, uveitis, uvetic macular edema, cyclitis, choroiditis, diffuse uveitis and scleritis.

As defined in the claims, the lipid nanoparticle comprises one or more cationic lipids, one or more non-cationic lipids and a PEG-modified lipid. In some embodiments, the lipid nanoparticle is a liposome. In some embodiments, the one or more cationic lipids is/are selected from the group consisting of cKK-E12, OF-02, C12-200, MC3, DLinDMA, DLinkC2DMA, ICE (Imidazol-based), HGT5000, HGT5001, HGT-5002, HGT4003, DODAC, DDAB, DMRIE, DOSPA, DOGS, DODAP, DODMA and DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, 3-(4-(bis(2-hydroxydodecyl)amino)butyl)-6-(4-((2-hydroxydodecyl)(2-hydroxyundecyl)amino)butyl)-1,4-dioxane-2,5-dione (Target 23), 3-(5-(bis(2-hydroxydodecyl)amino)pentan-2-yl)-6-(5-((2-hydroxydodecyl)(2-hydroxyundecyl)amino)pentan-2-yl)-1,4-dioxane-2,5-dione (Target 24), and combinations thereof.

In some embodiments, the one or more non-cationic lipids is/are selected from a group consisting of DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), and DOPG (,2-dioleoyl-sn-glycero-3-phospho-(1'-rac-glycerol)). In some embodiments, the PEG-modified lipid is selected from derivatized ceramides such as N-Octanoyl-Sphingosine-l-[Succinyl(Methoxy Polyethylene Glycol)-2000] (C8 PEG-2000 ceramide); PEG-modified lipids having a polyethylene glycol chain of up to 5 kDa in length covalently attached to a lipid with alkyl chain(s) of C₆-C₂₀ length, a PEGylated cholesterol and PEG-2K. In some embodiments, the cationic lipid constitutes about 30-70 % of the lipid nanoparticle by molar ratio. In some embodiments, the PEG-modified lipid comprises at least 1%, at least 2%, at least 3%, at least 4%, at least 5%, at least 6%, at least 7%, at least 8%, at least 9%, or at least 10% of the total lipids in the lipid nanoparticle.

In some embodiments, the lipid component of the lipid nanoparticle consists of a cationic lipid, a non-cationic lipid, cholesterol and a PEG-modified lipid. In some embodiments, the cationic lipid constitutes about 30-70 % of the lipid nanoparticle by molar ratio. In some embodiments, the PEG-modified lipid comprises at least 1%, at least 2%, at least 3%, at least 4%, at least 5%, at least 6%, at least 7%, at least 8%, at least 9%, or at least 10% of the total lipids in the lipid nanoparticle. In some embodiments, the ratio of cationic lipid(s) to non-cationic lipid(s) to cholesterol-based lipid(s) to PEG-modified lipid(s) is between about 30-60:25-35:20-30:1-15.

In one aspect, the invention provides a method of treating an ocular disease or disorder in a subject in need thereof, comprising: administering to an eye of the subject a composition comprising an effective amount of mRNA encoding a protein, wherein the mRNA is encapsulated in a lipid nanoparticle, and wherein administering the composition results in expression of the protein or the peptide encoded by the mRNA in one or more cells located in the outer nuclear layer (ONL), the inner segment photoreceptors (IS), the outer segment photoreceptors (OS), the retinal pigmented epithelium layer (RPE) of the retinal tissue, the choroid, and/or the sclera of the eye.

In some embodiments, the invention provides the mRNA is administered to the eye of the subject via intravitreal or suprachoroidal administration.

In some embodiments, the mRNA is administered to the eye of the subject via intravitreal administration. In some embodiments, the mRNA is administered to the eye of the subject via suprachoroidal administration. In some embodiments, the administering the composition results in expression of the protein or the peptide encoded by the mRNA in the retinal tissue. In some embodiments, the administering the composition results in expression of the protein or the peptide encoded by the mRNA in the choroid. In some embodiments, administering the composition results in expression of the protein or the peptide encoded by the mRNA in the sclera.

As defined in the claims, the subject is human. As defined in the claims, the effective amount of mRNA administered to the human subject ranges from 5 µg to 100 µg mRNA. In some embodiments, the effective amount of mRNA administered to the human subject ranges from 10 µg to 80 µg mRNA. In some embodiments, the effective amount of mRNA administered to the human subject ranges from 30 µg to 60 µg mRNA. In some embodiments, the composition is administered to the human subject by intravitreal injection. In some embodiments, the composition is administered to the human subject at a volume ranging from 30 µl about to about 100 µl.

As defined in the claims, the subject is suffering from a disease or disorder affecting the posterior retinal layers or a tissue of the posterior eye. As defined in the claims, the disease or disorder affecting the posterior retinal layers or the tissue of the posterior eye is selected from age-related macular degeneration (AMD), cytomegalovirus (CMV) retinitis, Leber's congenital amaurosis, Stargardt disease, Usher disease, chorioretinitis, retinal detachment, uveitis, uvetic macular edema, cyclitis, choroiditis, diffuse uveitis and scleritis.

As defined in the claims, the lipid nanoparticle comprises one or more cationic lipids, one or more non-cationic lipids and a PEG-modified lipid.

In some embodiments, the lipid nanoparticle comprises a cationic lipid selected from a group consisting of cKK-E12, OF-02, C12-200, MC3, DLinDMA, DLinkC2DMA, ICE (Imidazol-based), HGT5000, HGT5001, HGT-5002, HGT4003, DODAC, DDAB, DMRIE, DOSPA, DOGS, DODAP, DODMA and DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, 3-(4-(bis(2-hydroxydodecyl)amino)butyl)-6-(4-((2-hydroxydodecyl)(2-hydroxyundecyl)amino)butyl)-1,4-dioxane-2,5-dione (Target 23), 3-(5-(bis(2-hydroxydodecyl)amino)pentan-2-yl)-6-(5-((2-hydroxydodecyl)(2-hydroxyundecyl)amino)pentan-2-yl)-1,4-dioxane-2,5-dione (Target 24), and combinations thereof. In some embodiments, the lipid nanoparticle comprises a non-cationic lipid selected from a group consisting of DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine) and DOPG (,2-dioleoyl-sn-glycero-3-phospho-(1'-rac-glycerol)).

In some embodiments, the lipid nanoparticle comprises a PEG-modified lipid selected from derivatized ceramides such as N-Octanoyl-Sphingosine-1-[Succinyl(Methoxy Polyethylene Glycol)-2000] (C8 PEG-2000 ceramide); PEG-modified lipids having a polyethylene glycol chain of up to 5 kDa in length covalently attached to a lipid with alkyl chain(s) of C₆-C₂₀ length, a PEGylated cholesterol and PEG-2K.

In some embodiments, the lipid component of the lipid nanoparticle consists of a cationic lipid, a non-cationic lipid, cholesterol and a PEG-modified lipid.

In some embodiments, the cationic lipid constitutes about 30-70 % of the lipid nanoparticle by molar ratio. In some embodiments, the PEG-modified lipid comprises at least 1%, at least 2%, at least 3%, at least 4%, at least 5%, at least 6%, at least 7%, at least 8%, at least 9%, or at least 10% of the total lipids in the lipid nanoparticle. In some embodiments, the ratio of cationic lipid(s) to non-cationic lipid(s) to cholesterol-based lipid(s) to PEG-modified lipid(s) is between about 30-60:25-35:20-30:1-15.

In some embodiments, the mRNA encodes a protein or a peptide selected from a group consisting of an ocular protein or a peptide, a vaccine, an antibody or a fragment thereof, a hormone, a structural protein or peptide, an extracellular matrix protein or peptide, a vascular protein or peptide, an anti-tumor protein or peptide, an angiogenic protein or peptide, an anti-angiogenic protein or peptide, an antioxidant protein or peptide, a receptor protein or peptide, a signaling protein or peptide, a transcription factor and an enzyme.

In some embodiments, the mRNA encodes an ocular protein or a peptide selected from a group consisting of ADAM metallopeptidase domain 9, adhesins, ATP synthase, bestrophin 1, cadherins, chemokines, ciliary neurotrophic factor, collagens, complement factors, cytochromes, IGF, metalloproteinases, mitofusin, NADH dehydrogenase, OPA1, PDGF, peripherin 2, retinoschisin, SOD2, thrombospondin receptor, and vascular endothelial growth factor (VEGF).

In some embodiments, the mRNA encodes an antibody or a fragment thereof, that binds to ADAM metallopeptidase domain 9, adhesins, ATP synthase, bestrophin 1, cadherins, chemokines, ciliary neurotrophic factor, collagens, complement factors, cytochromes, IGF, metalloproteinases, mitofusin, NADH dehydrogenase, OPA1, PDGF, peripherin 2, retinoschisin, SOD2, thrombospondin receptor, or vascular endothelial growth factor (VEGF). In some embodiments, the mRNA encodes an antibody or a fragment thereof that binds to VEGF.

In some embodiments, the composition results in a decrease or amelioration of one or more symptoms associated with the ocular disease or disorder.

### BRIEF DESCRIPTION OF THE DRAWING

The drawings are for illustration purposes only not for limitation.

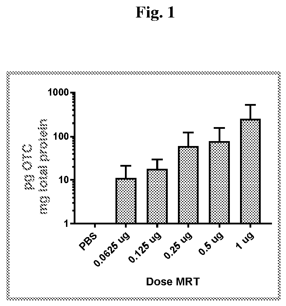

**Fig. 1** is a graph that depicts exemplary determination of the amount of exemplary protein expressed in the retinal tissue of mice at 24 hours after intravitreal administration of mRNA encoding the exemplary protein. The protein concentration was determined by ELISA assay.

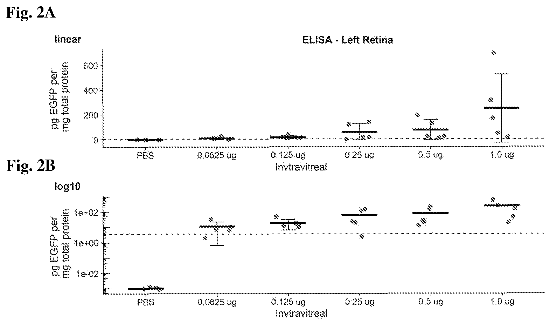

**Fig. 2A and 2B** are graphs that depict exemplary determination of the amount of EGFP protein expressed in the retinal tissue of mice at 24 hours after intravitreal administration of mRNA encoding EGFP. The protein was determined by ELISA assay. The data is represented in linear scale in Fig. 2A, and in logarithmic scale in Fig. 2B

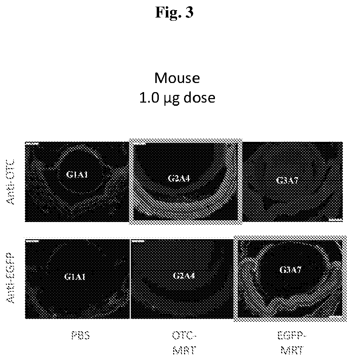

**Fig. 3** is a series of micrographs that depict exemplary detection and visualization of protein expressed in mouse retina tissue by immunostaining following intravitreal injection of mRNA. Upper panel shows immunostaining the retinal cross section using anti-OTC antibody. Lower panel shows immunostaining the retinal cross section using anti-EGFP antibody.

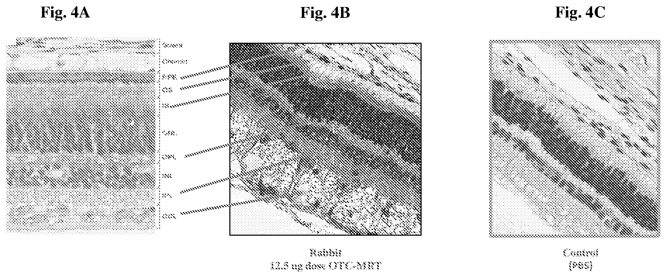

**Fig. 4A-C** are a series of micrographs depicts expression of mRNA encoded protein in retinal tissue in cross section of a rabbit eye. Fig 4A shows a schematic depiction of cross section of the retina, demarking the various tissue layers. Fig. 4B shows immunohistochemistry with positive staining for OTC in the layers indicated by arrows, indicating expression of OTC mRNA-encoded protein expression following intravitreal injection of the mRNA in rabbit eye.

### DEFINITIONS

In order for the present invention to be more readily understood, certain terms are first defined below. Additional definitions for the following terms and other terms are set forth throughout the specification.

*Alkyl:* As used herein, "alkyl" refers to a radical of a straight-chain or branched saturated hydrocarbon group having from 1 to 15 carbon atoms ("C₁₋₁₅ alkyl"). In some embodiments, an alkyl group has 1 to 3 carbon atoms ("C₁₋₃ alkyl"). Examples of C₁₋₃ alkyl groups include methyl (C₁), ethyl (C₂), n-propyl (C₃), and isopropyl (C₃). In some embodiments, an alkyl group has 8 to 12 carbon atoms ("C₈₋₁₂ alkyl"). Examples of C₈₋₁₂ alkyl groups include, without limitation, n-octyl (C₈), n-nonyl (C₉), n-decyl (C₁₀), n-undecyl (C₁₁), n-dodecyl (C₁₂) and the like. The prefix "n-" (normal) refers to unbranched alkyl groups. For example, *n*-C₈ alkyl refers to -(CH₂)₇CH₃, *n*-C₁₀ alkyl refers to -(CH₂)₉CH₃, etc.

*Amelioration:* As used herein, the term "amelioration" is meant the prevention, reduction or palliation of a state, or improvement of the state of a subject. Amelioration includes, but does not require complete recovery or complete prevention of a disease condition. In some embodiments, amelioration includes increasing levels of relevant protein or its activity that is deficient in relevant disease tissues.

*Amino acid:* As used herein, term "amino acid," in its broadest sense, refers to any compound and/or substance that can be incorporated into a polypeptide chain. In some embodiments, an amino acid has the general structure H₂N-C(H)(R)-COOH. In some embodiments, an amino acid is a naturally occurring amino acid. In some embodiments, an amino acid is a synthetic amino acid; in some embodiments, an amino acid is a d-amino acid; in some embodiments, an amino acid is an l-amino acid. "Standard amino acid" refers to any of the twenty standard l-amino acids commonly found in naturally occurring peptides. "Nonstandard amino acid" refers to any amino acid, other than the standard amino acids, regardless of whether it is prepared synthetically or obtained from a natural source. As used herein, "synthetic amino acid" encompasses chemically modified amino acids, including but not limited to salts, amino acid derivatives (such as amides), and/or substitutions. Amino acids, including carboxy- and/or amino-terminal amino acids in peptides, can be modified by methylation, amidation, acetylation, protecting groups, and/or substitution with other chemical groups that can change the peptide's circulating half-life without adversely affecting their activity. Amino acids may participate in a disulfide bond. Amino acids may comprise one or posttranslational modifications, such as association with one or more chemical entities (*e.g.,* methyl groups, acetate groups, acetyl groups, phosphate groups, formyl moieties, isoprenoid groups, sulfate groups, polyethylene glycol moieties, lipid moieties, carbohydrate moieties, biotin moieties, *etc*.). The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Animal:* As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e.g.,* a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.

*Approximately or about:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Biologically active:* As used herein, the phrase "biologically active" refers to a characteristic of any agent that has activity in a biological system, and particularly in an organism. For instance, an agent that, when administered to an organism, has a biological effect on that organism, is considered to be biologically active. In particular embodiments, where a protein or polypeptide is biologically active, a portion of that protein or polypeptide that shares at least one biological activity of the protein or polypeptide is typically referred to as a "biologically active" portion.

*Delivery:* As used herein, the term "delivery" encompasses both local and systemic delivery. For example, delivery of mRNA encompasses situations in which an mRNA is delivered to a target tissue and the encoded protein is expressed and retained within the target tissue (also referred to as "local distribution" or "local delivery"), and situations in which an mRNA is delivered to a target tissue and the encoded protein is expressed and secreted into patient's circulation system (e.g., serum) and systematically distributed and taken up by other tissues (also referred to as "systemic distribution" or "systemic delivery).

*Expression:* As used herein, "expression" of a nucleic acid sequence refers to one or more of the following events: (1) production of an RNA template from a DNA sequence (*e.g*., by transcription); (2) processing of an RNA transcript *(*e*.g.,* by splicing, editing, 5' cap formation, and/or 3' end formation); (3) translation of an RNA into a polypeptide or protein; and/or (4) post-translational modification of a polypeptide or protein. In this application, the terms "expression" and "production," and grammatical equivalent, are used inter-changeably.

*Fragment*: The term "fragment" as used herein refers to polypeptides and is defined as any discrete portion of a given polypeptide that is unique to or characteristic of that polypeptide. The term as used herein also refers to any discrete portion of a given polypeptide that retains at least a fraction of the activity of the full-length polypeptide. Preferably the fraction of activity retained is at least 10% of the activity of the full-length polypeptide. More preferably the fraction of activity retained is at least 20%, 30%, 40%, 50%, 60%, 70%, 80% or 90% of the activity of the full-length polypeptide. More preferably still the fraction of activity retained is at least 95%, 96%, 97%, 98% or 99% of the activity of the full-length polypeptide. Most preferably, the fraction of activity retained is 100% of the activity of the full-length polypeptide. The term as used herein also refers to any portion of a given polypeptide that includes at least an established sequence element found in the full-length polypeptide. Preferably, the sequence element spans at least 4-5, more preferably at least about 10, 15, 20, 25, 30, 35, 40, 45, 50 or more amino acids of the full-length polypeptide.

*Functional*: As used herein, a "functional" biological molecule is a biological molecule in a form in which it exhibits a property and/or activity by which it is characterized.

*Half-life:* As used herein, the term "half-life" is the time required for a quantity such as nucleic acid or protein concentration or activity to fall to half of its value as measured at the beginning of a time period.

*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

*In Vitro*: As used herein, the term *"in vitro"* refers to events that occur in an artificial environment, *e.g.,* in a test tube or reaction vessel, in cell culture, *etc.,* rather than within a multicellular organism.

*In Vivo*: As used herein, the term *"in vivo"* refers to events that occur within a multicellular organism, such as a human and a non-human animal. In the context of cell-based systems, the term may be used to refer to events that occur within a living cell (as opposed to, for example, *in vitro* systems).

*Isolated:* As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from about 10%, about 20%, about 30%, about 40%, about 50%, about 60%, about 70%, about 80%, about 90%, about 91%, about 92%, about 93%, about 94%, about 95%, about 96%, about 97%, about 98%, about 99%, or more than about 99% of the other components with which they were initially associated. In some embodiments, isolated agents are about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components. As used herein, calculation of percent purity of isolated substances and/or entities should not include excipients (e.g., buffer, solvent, water, etc.).

*messenger RNA (mRNA):* As used herein, the term "messenger RNA (mRNA)" refers to a polynucleotide that encodes at least one polypeptide. mRNA as used herein encompasses both modified and unmodified RNA. mRNA may contain one or more coding and non-coding regions. mRNA can be purified from natural sources, produced using recombinant expression systems and optionally purified, chemically synthesized, *etc.* Where appropriate, *e.g.,* in the case of chemically synthesized molecules, mRNA can comprise nucleoside analogs such as analogs having chemically modified bases or sugars, backbone modifications, *etc.* An mRNA sequence is presented in the 5' to 3' direction unless otherwise indicated. In some embodiments, an mRNA is or comprises natural nucleosides (*e.g.,* adenosine, guanosine, cytidine, uridine); nucleoside analogs (*e*.*g*., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g.,* methylated bases); intercalated bases; modified sugars (*e.g.,* 2'-fluororibose, ribose, 2'-deoxyribose, arabinose, and hexose); and/or modified phosphate groups (*e.g.,* phosphorothioates and 5'-N-phosphoramidite linkages).

*Nucleic acid:* As used herein, the term "nucleic acid," in its broadest sense, refers to any compound and/or substance that is or can be incorporated into a polynucleotide chain. In some embodiments, a nucleic acid is a compound and/or substance that is or can be incorporated into a polynucleotide chain via a phosphodiester linkage. In some embodiments, "nucleic acid" refers to individual nucleic acid residues (*e.g.,* nucleotides and/or nucleosides). In some embodiments, "nucleic acid" refers to a polynucleotide chain comprising individual nucleic acid residues. In some embodiments, "nucleic acid" encompasses RNA as well as single and/or double-stranded DNA and/or cDNA.

*Patient:* As used herein, the term "patient" or "subject" refers to any organism to which a provided composition may be administered, *e.g.,* for experimental, diagnostic, prophylactic, cosmetic, and/or therapeutic purposes. Typical patients include animals (*e.g.,* mammals such as mice, rats, rabbits, non-human primates, and/or humans). In some embodiments, a patient is a human. A human includes pre and post-natal forms.

*Pharmaceutically acceptable:* The term "pharmaceutically acceptable" as used herein, refers to substances that, within the scope of sound medical judgment, are suitable for use in contact with the tissues of human beings and animals without excessive toxicity, irritation, allergic response, or other problem or complication, commensurate with a reasonable benefit/risk ratio.

*Pharmaceutically acceptable salt:* Pharmaceutically acceptable salts are well known in the art. For example, S. M. Berge et al., describes pharmaceutically acceptable salts in detail in J. Pharmaceutical Sciences (1977) 66:1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2- naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄ alkyl)₄ salts. Representative alkali or alkaline earth metal salts include sodium, lithium, potassium, calcium, magnesium, and the like. Further pharmaceutically acceptable salts include, when appropriate, nontoxic ammonium quaternary ammonium, and amine cations formed using counterions such as halide, hydroxide, carboxylate, sulfate, phosphate, nitrate, sulfonate and aryl sulfonate. Further pharmaceutically acceptable salts include salts formed from the quarternization of an amine using an appropriate electrophile, e.g., an alkyl halide, to form a quarternized alkylated amino salt.

*Subject:* As used herein, the term "subject" refers to a human or any non-human animal (e.g., mouse, rat, rabbit, dog, cat, cattle, swine, sheep, horse or primate). A human includes pre- and post-natal forms. As defined in the claims, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Therapeutically effective amount*: As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.

*Treatment*: As used herein, the term "treatment" (also "treat" or "treating") refers to any administration of a substance (*e.g.,* provided compositions) that partially or completely alleviates, ameliorates, relives, inhibits, delays onset of, reduces severity of, and/or reduces incidence of one or more symptoms, features, and/or causes of a particular disease, disorder, and/or condition (*e.g.,* influenza). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

### DETAILED DESCRIPTION

The present invention provides, among other things, methods and compositions for treating ocular diseases, disorders or conditions based on mRNA therapy, as defined in the claims. In particular, the present invention provides methods for treating ocular diseases, disorders or conditions by administering to a subject in need of treatment a composition comprising an mRNA. mRNA may be administered naked, or encapsulated within a nanoparticle. Suitable nanoparticle may be lipid or polymer based. In some embodiments, a suitable nanoparticle for mRNA delivery is a liposome. As used herein, the term "liposome" refers to any lamellar, multilamellar, or solid nanoparticle vesicle. Typically, a liposome as used herein can be formed by mixing one or more lipids or by mixing one or more lipids and polymer(s). Thus, the term "liposome" as used herein encompasses both lipid and polymer based nanoparticles. In some embodiments, a liposome suitable for the present invention contains cationic or non-cationic lipid(s), cholesterol-based lipid(s) and PEG-modified lipid(s).

### Ocular diseases, disorders or conditions

The present invention may be used to treat a subject who is suffering from or susceptible to an ocular disease, disorder or condition. As used herein, an "ocular disease, disorder or condition" refers to a disease, disorder or condition affecting the eye and/or vision. Ocular diseases, disorders or conditions can affect one or more of the following parts of the eye: eyelid, lacrimal system and orbit; conjunctiva; sclera, cornea, iris and cilliary body; lens; choroid and retina; vitreous body and globe; optic nerve and visual pathways; and ocular muscles. In some embodiments, an ocular disease, disorder or condition may be caused by a protein deficiency or dysfunctions in the eye or parts of the anatomy associated with vision. In some embodiments, an ocular disease, disorder or condition may be caused by a protein surplus, over expression, and/or over activation in the eye or parts of the anatomy associated with vision.

Exemplary ocular diseases, disorders or conditions include, but are not limited to, age-related macular degeneration (AMD), cystoid cytomegalovirus (CMV) retinitis, scleritis, and uveitis.

In various embodiments, the present invention may be used to deliver an mRNA encoding a protein that is deficient in any of the ocular diseases, disorders or conditions described herein. In such embodiments, the delivery of mRNA typically results in increased protein expression and/or activity in the eye sufficient to treat protein deficiency. In some embodiments, an mRNA suitable for the invention may encode a wild-type or naturally occurring protein sequence. In some embodiments, an mRNA suitable for the invention may be a wild-type or naturally occurring sequence. In some embodiments, the mRNA suitable for the invention may be a codon-optimized sequence. In some embodiments, an mRNA suitable for the invention may encode an amino acid sequence having substantial homology or identity to the wild-type or naturally-occurring amino acid protein sequence (e.g., having at least 50%, 55%, 60%, 65%, 70%, 75%, 80%, 85%, 90%, 95%, 98% sequence identity to the wild-type or naturally-occurring sequence).

Mutations in more than 40 genes result in Retinitis pigmentosa (RP). For example, mutations in the ABCA4 gene are associated with Stargardt's disease, a Retinoschisin gene is mutated in Hereditary retinoschisis, the RPE65 gene is mutated in Leber's congenital amaurosis (LCA), Mitochondrial DNA mutations in ND1, ND4 or ND6 genes are found in Leber's hereditary optic neuropathy and the Myo7 gene is mutated in Usher disease.

In some embodiments, the present invention may be used to deliver an mRNA encoding a therapeutic agent that inhibits, down-regulates, reduces a protein expression and/or activity, the excess level of which is associated with an ocular disease, disorder or condition. Such a therapeutic agent may be a peptide, an antibody or other polypeptides or proteins.

In some embodiments, the present invention may be used to deliver an mRNA encoding an antibody, a soluble receptor or other binding protein. Typically, a suitable mRNA encodes an antibody that inhibits, down-regulates, or reduces a protein that is present in excess in amount and/or activity in an ocular disease, disorder or condition. In some embodiments, a suitable mRNA encodes an antibody that activates, up-regulates or increases a protein activity that is deficient in an ocular disease, disorder or condition. Suitable exemplary antibodies encoded by mRNAs according to the present invention include, but are not limited to, antibodies against VEGF, TNFα, IL-6, ICAM-1, VCAM-1, or soluble receptors such as VEGF receptors (e.g., VEGFR1).

In some embodiments, the compositions of the invention comprise one or more mRNAs encoding an antibody against VEGF or a VEGF receptor. Such compositions may be used to treat diseases or disorders affecting the eye that are ameliorated by neutralizing VEGF or blocking VEGF signaling. Such diseases or disorders include macular degeneration (including age related macular degeneration (AMD)), branch retinal vein occlusion (BRVO), central retinal vein occlusion (CRVO), diabetic macular edema (DME), cystoid macular edema (CME), familial exudative viteoretinopathy, retinal angiogenesis and Coats' disease.

In other embodiments, the compositions of the invention comprise one or more mRNAs encoding antibody against pro-inflammatory cytokines including IL-1β, IL-6, IL-17A, and TNF-α. Such compositions may be used to treat diseases or disorders affecting the eye which are caused by an inflammatory conditions. Such diseases or disorders include scleretis, uveitis (including diffuse uveitis), glaucoma, dry eye syndrome, cyclitis, choroiditis and retinitis. For example, uveitic macular edema may benefit from therapy with an anti-TNF-α antibody or an anti-TNFR receptor antibody.

As used herein, the term "antibody" encompasses both intact antibody and antibody fragment. Typically, an intact "antibody" is an immunoglobulin that binds specifically to a particular antigen. An antibody may be a member of any immunoglobulin class, including any of the human classes: IgG, IgM, IgE, IgA, and IgD. Typically, an intact antibody is a tetramer. Each tetramer is composed of two identical pairs of polypeptide chains, each pair having one "light" (approximately 25 kD) and one "heavy" chain (approximately 50-70 kD). The N-terminus of each chain defines a variable region of about 100 to 110 or more amino acids primarily responsible for antigen recognition. The terms "variable light chain" (VL) and "variable heavy chain" (VH) refer to these corresponding regions on the light and heavy chain respectively. Each variable region can be further subdivided into hypervariable (HV) and framework (FR) regions. The hypervariable regions comprise three areas of hypervariability sequence called complementarity determining regions (CDR 1, CDR 2 and CDR 3), separated by four framework regions (FR1, FR2, FR2, and FR4) which form a beta-sheet structure and serve as a scaffold to hold the HV regions in position. The C-terminus of each heavy and light chain defines a constant region consisting of one domain for the light chain (CL) and three for the heavy chain (CH1, CH2 and CH3). A light chain of immunoglobulins can be further differentiated into the isotypes *kappa* and *lamda.*

In some embodiments, the terms "intact antibody" or "fully assembled antibody" are used in reference to an antibody that contains two heavy chains and two light chains, optionally associated by disulfide bonds as occurs with naturally-produced antibodies. In some embodiments, an antibody according to the present invention is an antibody fragment.

In some embodiments, the present invention can be used to deliver an "antibody fragment." As used herein, an "antibody fragment" includes a portion of an intact antibody, such as, for example, the antigen-binding or variable region of an antibody. Examples of antibody fragments include Fab, Fab', F(ab')₂, and Fv fragments; triabodies; tetrabodies; linear antibodies; single-chain antibody molecules; and multi specific antibodies formed from antibody fragments. For example, antibody fragments include isolated fragments, "Fv" fragments, consisting of the variable regions of the heavy and light chains, recombinant single chain polypeptide molecules in which light and heavy chain variable regions are connected by a peptide linker ("ScFv proteins"), and minimal recognition units consisting of the amino acid residues that mimic the hypervariable region. In many embodiments, an antibody fragment contains a sufficient sequence of the parent antibody of which it is a fragment that it binds to the same antigen as does the parent antibody; in some embodiments, a fragment binds to the antigen with a comparable affinity to that of the parent antibody and/or competes with the parent antibody for binding to the antigen. Examples of antigen binding fragments of an antibody include, but are not limited to, Fab fragment, Fab' fragment, F(ab')₂ fragment, scFv fragment, Fv fragment, dsFv diabody, dAb fragment, Fd' fragment, Fd fragment, and an isolated complementarity determining region (CDR). Suitable antibodies include monoclonal antibodies, polyclonal antibodies, antibody mixtures or cocktails, human or humanized antibodies, chimeric antibodies, or bi-specific antibodies.

### mRNA Synthesis

mRNAs according to the present invention may be synthesized according to any of a variety of known methods. For example, mRNAs according to the present invention may be synthesized via *in vitro* transcription (IVT). Briefly, IVT is typically performed with a linear or circular DNA template containing a promoter, a pool of ribonucleotide triphosphates, a buffer system that may include DTT and magnesium ions, and an appropriate RNA polymerase (e.g., T3, T7 or SP6 RNA polymerase), DNAse I, pyrophosphatase, and/or RNAse inhibitor. The exact conditions will vary according to the specific application.

In some embodiments, for the preparation of mRNA according to the invention, a DNA template is transcribed *in vitro.* A suitable DNA template typically has a promoter, for example a T3, T7 or SP6 promoter, for *in vitro* transcription, followed by desired nucleotide sequence for desired mRNA and a termination signal.

Desired mRNA sequence(s) may be determined and incorporated into a DNA template using standard methods. For example, the mRNA suitable for the invention may be a codon-optimized sequence. As used herein, the terms "codon optimization" and "codon-optimized" refer to modifications of the codon composition of a naturally-occurring or wild-type nucleic acid encoding a peptide, polypeptide or protein that do not alter its amino acid sequence, thereby improving protein expression of said nucleic acid. Optimization algorithms may then be used for selection of suitable codons. Typically, the G/C content can be optimized to achieve the highest possible G/C content, to adjust codon usage to avoid rare or rate-limiting codons, to remove destabilizing nucleic acid sequences or motifs and/or to eliminate pause sites or terminator sequences. The optimized RNA sequence can be established and displayed, for example, with the aid of an appropriate display device and compared with the original (wild-type) sequence. A secondary structure can also be analyzed to calculate stabilizing and destabilizing properties or, respectively, regions of the RNA.

### Modified mRNA

In some embodiments, mRNA according to the present invention may be synthesized as unmodified or modified mRNA. In specific embodiments, an mRNA for use with the invention comprises or consists of naturally-occurring nucleosides (or unmodified nucleosides; *i.e.,* adenosine, guanosine, cytidine, and uridine). In other embodiments, mRNAs for use with the invention are modified to enhance stability. Modifications of mRNA can include, for example, modifications of the nucleotides of the RNA. A modified mRNA according to the invention can thus include, for example, backbone modifications, sugar modifications or base modifications. In some embodiments, mRNAs may be synthesized from naturally occurring nucleotides and/or nucleotide analogues (modified nucleotides) including, but not limited to, purines (adenine (A), guanine (G)) or pyrimidines (thymine (T), cytosine (C), uracil (U)), and as modified nucleotides analogues or derivatives of purines and pyrimidines, such as e.g. 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, .beta.-D-mannosyl-queosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art e.g. from the U.S. Pat. No. 4,373,071, U.S. Pat. No. 4,401,796, U.S. Pat. No. 4,415,732, U.S. Pat. No. 4,458,066, U.S. Pat. No. 4,500,707, U.S. Pat. No. 4,668,777, U.S. Pat. No. 4,973,679, U.S. Pat. No. 5,047,524, U.S. Pat. No. 5,132,418, U.S. Pat. No. 5,153,319, U.S. Pat. Nos. 5,262,530 and 5,700,642.

In some embodiments, mRNAs may contain RNA backbone modifications. Typically, a backbone modification is a modification in which the phosphates of the backbone of the nucleotides contained in the RNA are modified chemically. Exemplary backbone modifications typically include, but are not limited to, modifications from the group consisting of methylphosphonates, methylphosphoramidates, phosphoramidates, phosphorothioates (e.g. cytidine 5'-O-(1-thiophosphate)), boranophosphates, positively charged guanidinium groups etc., which means by replacing the phosphodiester linkage by other anionic, cationic or neutral groups.

In some embodiments, mRNAs may contain sugar modifications. A typical sugar modification is a chemical modification of the sugar of the nucleotides it contains including, but not limited to, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

In some embodiments, mRNAs may contain modifications of the bases of the nucleotides (base modifications). A modified nucleotide which contains a base modification is also called a base-modified nucleotide. Examples of such base-modified nucleotides include, but are not limited to, 2-amino-6-chloropurine riboside 5'-triphosphate, 2-aminoadenosine 5'-triphosphate, 2-thiocytidine 5'-triphosphate, 2-thiouridine 5'-triphosphate, 4-thiouridine 5'-triphosphate, 5-aminoallylcytidine 5'-triphosphate, 5-aminoallyluridine 5'-triphosphate, 5-bromocytidine 5'-triphosphate, 5-bromouridine 5'-triphosphate, 5-iodocytidine 5'-triphosphate, 5-iodouridine 5'-triphosphate, 5-methylcytidine 5'-triphosphate, 5-methyluridine 5'-triphosphate, 6-azacytidine 5'-triphosphate, 6-azauridine 5'-triphosphate, 6-chloropurine riboside 5'-triphosphate, 7-deazaadenosine 5'-triphosphate, 7-deazaguanosine 5'-triphosphate, 8-azaadenosine 5'-triphosphate, 8-azidoadenosine 5'-triphosphate, benzimidazole riboside 5'-triphosphate, N1-methyladenosine 5'-triphosphate, N1-methylguanosine 5'-triphosphate, N6-methyladenosine 5'-triphosphate, O6-methylguanosine 5'-triphosphate, pseudouridine 5'-triphosphate, puromycin 5'-triphosphate or xanthosine 5'-triphosphate.

Typically, mRNA synthesis includes the addition of a "cap" on the N-terminal (5') end, and a "tail" on the C-terminal (3') end. The presence of the cap is important in providing resistance to nucleases found in most eukaryotic cells. The presence of a "tail" serves to protect the mRNA from exonuclease degradation.

### Cap structure

In some embodiments, mRNAs include a 5' cap structure. A 5' cap is typically added as follows: first, an RNA terminal phosphatase removes one of the terminal phosphate groups from the 5' nucleotide, leaving two terminal phosphates; guanosine triphosphate (GTP) is then added to the terminal phosphates via a guanylyl transferase, producing a 5'5'5 triphosphate linkage; and the 7-nitrogen of guanine is then methylated by a methyltransferase. Examples of cap structures include, but are not limited to, m7G(5')ppp (5'(A,G(5')ppp(5')A and G(5')ppp(5')G.

Naturally occurring cap structures comprise a 7-methyl guanosine that is linked via a triphosphate bridge to the 5'-end of the first transcribed nucleotide, resulting in a dinucleotide cap of m⁷G(5')ppp(5')N, where N is any nucleoside. *In vivo,* the cap is added enzymatically. The cap is added in the nucleus and is catalyzed by the enzyme guanylyl transferase. The addition of the cap to the 5' terminal end of RNA occurs immediately after initiation of transcription. The terminal nucleoside is typically a guanosine, and is in the reverse orientation to all the other nucleotides, i.e., G(5')ppp(5')GpNpNp.

A common cap for mRNA produced by *in vitro* transcription is m⁷G(5')ppp(5')G, which has been used as the dinucleotide cap in transcription with T7 or SP6 RNA polymerase *in vitro* to obtain RNAs having a cap structure in their 5'-termini. The prevailing method for the *in vitro* synthesis of capped mRNA employs a pre-formed dinucleotide of the form m⁷G(5')ppp(5')G ("m⁷GpppG") as an initiator of transcription.

To date, a usual form of a synthetic dinucleotide cap used in *in vitro* translation experiments is the Anti-Reverse Cap Analog ("ARCA") or modified ARCA, which is generally a modified cap analog in which the 2' or 3' OH group is replaced with -OCH₃.

Additional cap analogs include, but are not limited to, a chemical structures selected from the group consisting of m⁷GpppG, m⁷GpppA, m⁷GpppC; unmethylated cap analogs (e.g., GpppG); dimethylated cap analog (e.g., m^{2,7}GpppG), trimethylated cap analog (e.g., m^{2,2,7}GpppG), dimethylated symmetrical cap analogs (e.g., m⁷Gpppm⁷G), or anti reverse cap analogs (e.g., ARCA; m⁷,^{2'Ome}GpppG, M^{72'd}GppppG, m^{7,3'Ome}GpppG, m^{7,3'd}GpppG and their tetraphosphate derivatives) (see, e.g., Jemielity, J. et al., "Novel 'anti-reverse' cap analogs with superior translational properties", RNA, 9: 1108-1122 (2003)).

In some embodiments, a suitable cap is a 7-methyl guanylate ("m⁷G") linked via a triphosphate bridge to the 5'-end of the first transcribed nucleotide, resulting in m⁷G(5')ppp(5')N, where N is any nucleoside. A preferred embodiment of a m⁷G cap utilized in embodiments of the invention is m⁷G(5')ppp(5')G.

In some embodiments, the cap is a CapO structure. CapO structures lack a 2'-O-methyl residue of the ribose attached to bases 1 and 2. In some embodiments, the cap is a Cap1 structure. Cap1 structures have a 2'-O-methyl residue at base 2. In some embodiments, the cap is a Cap2 structure. Cap2 structures have a 2'-O-methyl residue attached to both bases 2 and 3.

A variety of m⁷G cap analogs are known in the art, many of which are commercially available. These include the m⁷GpppG described above, as well as the ARCA 3'-OCH₃ and 2'-OCH₃ cap analogs (Jemielity, J. et al., RNA, 9: 1108-1122 (2003)). Additional cap analogs for use in embodiments of the invention include N7-benzylated dinucleoside tetraphosphate analogs (described in Grudzien, E. et al., RNA, 10: 1479-1487 (2004)), phosphorothioate cap analogs (described in Grudzien-Nogalska, E., et al., RNA, 13: 1745-1755 (2007)), and cap analogs (including biotinylated cap analogs) described in U.S. Patent Nos. 8,093,367 and 8,304,529.

### Tail structure

Typically, the presence of a "tail" serves to protect the mRNA from exonuclease degradation. The poly A tail is thought to stabilize natural messengers and synthetic sense RNA. Therefore, in certain embodiments a long poly A tail can be added to an mRNA molecule thus rendering the RNA more stable. Poly A tails can be added using a variety of art-recognized techniques. For example, long poly A tails can be added to synthetic or *in vitro* transcribed RNA using poly A polymerase (Yokoe, et al. Nature Biotechnology. 1996; 14: 1252-1256). A transcription vector can also encode long poly A tails. In addition, poly A tails can be added by transcription directly from PCR products. Poly A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1991 edition)).

In some embodiments, mRNAs include a 3' poly(A) tail structure. Typically, the length of the poly A tail can be at least about 10, 50, 100, 200, 300, 400 at least 500 nucleotides. In some embodiments, a poly-A tail on the 3' terminus of mRNA typically includes about 10 to 300 adenosine nucleotides (*e.g.,* about 10 to 200 adenosine nucleotides, about 10 to 150 adenosine nucleotides, about 10 to 100 adenosine nucleotides, about 20 to 70 adenosine nucleotides, or about 20 to 60 adenosine nucleotides). In some embodiments, a poly(U) tail may be used to instead of a poly(A) tail described herein. In some embodiments, a poly(U) tail may be added to a poly(A) tail described herein. In some embodiments, mRNAs include a 3' poly(C) tail structure. A suitable poly(C) tail on the 3' terminus of mRNA typically include about 10 to 200 cytosine nucleotides (*e.g.,* about 10 to 150 cytosine nucleotides, about 10 to 100 cytosine nucleotides, about 20 to 70 cytosine nucleotides, about 20 to 60 cytosine nucleotides, or about 10 to 40 cytosine nucleotides). The poly(C) tail may be added to a poly(A) and/or poly(U) tail or may substitute the poly(A) and/or poly(U) tail.

In some embodiments, the length of the poly(A), poly(U) or poly(C) tail is adjusted to control the stability of a modified sense mRNA molecule of the invention and, thus, the transcription of protein. For example, since the length of a tail structure can influence the half-life of a sense mRNA molecule, the length of the tail can be adjusted to modify the level of resistance of the mRNA to nucleases and thereby control the time course of polynucleotide expression and/or polypeptide production in a target cell.

### 5' and 3' Untranslated Region

In some embodiments, mRNAs include a 5' and/or 3' untranslated region. In some embodiments, a 5' untranslated region includes one or more elements that affect an mRNA's stability or translation, for example, an iron responsive element. In some embodiments, a 5' untranslated region may be between about 50 and 500 nucleotides in length.

In some embodiments, a 3' untranslated region includes one or more of a polyadenylation signal, a binding site for proteins that affect an mRNA's stability of location in a cell, or one or more binding sites for miRNAs. In some embodiments, a 3' untranslated region may be between 50 and 500 nucleotides in length or longer.

Exemplary 3' and/or 5' UTR sequences can be derived from mRNA molecules which are stable (e.g., globin, actin, GAPDH, tubulin, histone, or citric acid cycle enzymes) to increase the stability of the sense mRNA molecule. For example, a 5' UTR sequence may include a partial sequence of a CMV immediate-early 1 (IE1) gene, or a fragment thereof to improve the nuclease resistance and/or improve the half-life of the polynucleotide. Also contemplated is the inclusion of a sequence encoding human growth hormone (hGH), or a fragment thereof to the 3' end or untranslated region of the polynucleotide (e.g., mRNA) to further stabilize the polynucleotide. Generally, these modifications improve the stability and/or pharmacokinetic properties (e.g., half-life) of the polynucleotide relative to their unmodified counterparts, and include, for example modifications made to improve such polynucleotides' resistance to *in vivo* nuclease digestion.

### Delivery Vehicles

mRNA described herein may be delivered as naked RNA (unpackaged) or via delivery vehicles. As used herein, the terms "delivery vehicle," "transfer vehicle," "nanoparticle" or grammatical equivalent, are used interchangeably.

In some embodiments, mRNAs may be delivered via a single delivery vehicle. In some embodiments, mRNAs may be delivered via one or more delivery vehicles each of a different composition.

### Liposomal delivery vehicles

As defined in the claims, a suitable delivery vehicle is a lipid nanoparticle. As used herein, lipid nanoparticles, are usually characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol., 16: 307-321, 1998). Bilayer membranes of the liposomes can also be formed by amphiphilic polymers and surfactants (e.g., polymerosomes, niosomes, etc.). In the context of the present invention, a liposomal delivery vehicle typically serves to transport a desired mRNA to a target cell or tissue. The process of incorporation of a desired mRNA into a liposome is often referred to as "loading". Exemplary methods are described in Lasic, et al., FEBS Lett., 312: 255-258, 1992. The liposome-incorporated nucleic acids may be completely or partially located in the interior space of the liposome, within the bilayer membrane of the liposome, or associated with the exterior surface of the liposome membrane. The incorporation of a nucleic acid into liposomes is also referred to herein as "encapsulation" wherein the nucleic acid is entirely contained within the interior space of the liposome. The purpose of incorporating an mRNA into a transfer vehicle, such as a liposome, is often to protect the nucleic acid from an environment which may contain enzymes or chemicals that degrade nucleic acids and/or systems or receptors that cause the rapid excretion of the nucleic acids. Accordingly, in some embodiments, a suitable delivery vehicle is capable of enhancing the stability of the mRNA contained therein and/or facilitate the delivery of mRNA to the target cell or tissue.

In some embodiments, a nanoparticle delivery vehicle is a liposome. In some embodiments, a liposome comprises one or more cationic lipids, one or more non-cationic lipids, one or more cholesterol-based lipids, or one or more PEG-modified lipids. A typical liposome for use with the invention is composed of four lipid components: a cationic lipid, a non-cationic lipid (e.g., DOPE or DEPE), a cholesterol-based lipid (e.g., cholesterol) and a PEG-modified lipid (e.g., DMG-PEG2K). In some embodiments, a liposome comprises no more than three distinct lipid components. In some embodiments, one distinct lipid component is a sterol-based cationic lipid. An exemplary liposome is composed of three lipid components: a sterol-based cationic lipid, a non-cationic lipid (e.g., DOPE or DEPE) and a PEG-modified lipid (e.g., DMG-PEG2K).

### Cationic Lipids

As used herein, the phrase "cationic lipids" refers to any of a number of lipid species that have a net positive charge at a selected pH, such as physiological pH.

Suitable cationic lipids for use in the compositions and methods of the invention include the cationic lipids as described in International Patent Publication WO 2010/144740. In certain embodiments, the compositions and methods of the present invention include a cationic lipid, (6Z,9Z,28Z,31Z)-heptatriaconta-6,9,28,31-tetraen-19-yl 4-(dimethylamino) butanoate, having a compound structure of: 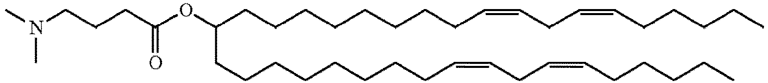 and pharmaceutically acceptable salts thereof.

Other suitable cationic lipids for use in the compositions and methods of the present invention include ionizable cationic lipids as described in International Patent Publication WO 2013/149140. In some embodiments, the compositions and methods of the present invention include a cationic lipid of one of the following formulas: 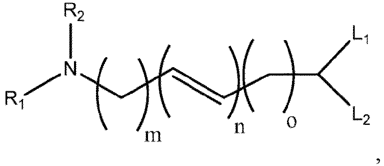 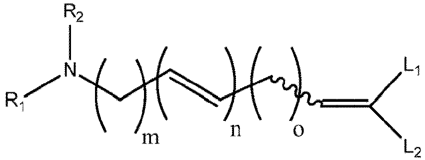 or a pharmaceutically acceptable salt thereof, wherein R₁ and R₂ are each independently selected from the group consisting of hydrogen, an optionally substituted, variably saturated or unsaturated C₁-C₂₀ alkyl and an optionally substituted, variably saturated or unsaturated C₆-C₂₀ acyl; wherein L₁ and L₂ are each independently selected from the group consisting of hydrogen, an optionally substituted C₁-C₃₀ alkyl, an optionally substituted variably unsaturated C₁-C₃₀ alkenyl, and an optionally substituted C₁-C₃₀ alkynyl; wherein m and o are each independently selected from the group consisting of zero and any positive integer (e.g., where m is three); and wherein n is zero or any positive integer (e.g., where n is one). In certain embodiments, the compositions and methods of the present invention include the cationic lipid (15Z, 18Z)-N,N-dimethyl-6-(9Z,12Z)-octadeca-9,12-dien-1-yl) tetracosa-15,18-dien-1-amine ("HGT5000"), having a compound structure of: 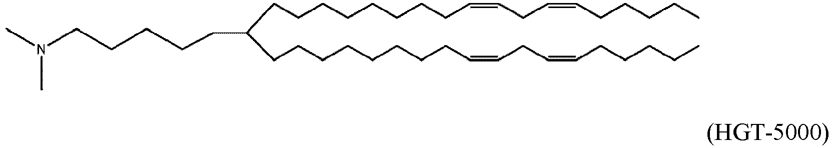 and pharmaceutically acceptable salts thereof. In certain embodiments, the compositions and methods of the present invention include the cationic lipid (15Z, 18Z)-N,N-dimethyl-6-((9Z,12Z)-octadeca-9,12-dien-1-yl) tetracosa-4,15,18-trien-1-amine ("HGT5001"), having a compound structure of: 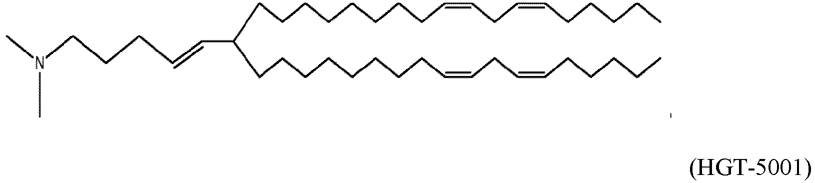 and pharmaceutically acceptable salts thereof. In certain embodiments, the compositions and methods of the present invention include the cationic lipid and (15Z,18Z)-N,N-dimethyl-6-((9Z,12Z)-octadeca-9,12-dien-1-yl) tetracosa-5,15,18-trien- 1 -amine ("HGT5002"), having a compound structure of: 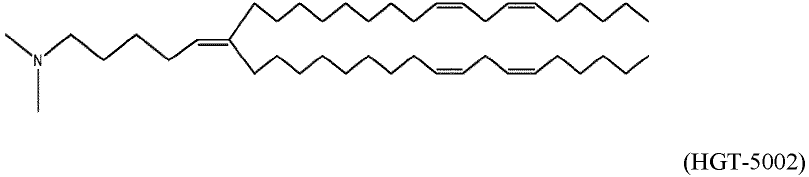 and pharmaceutically acceptable salts thereof.

Other suitable cationic lipids for use in the compositions and methods of the invention include cationic lipids described as aminoalcohol lipidoids in International Patent Publication WO 2010/053572. In certain embodiments, the compositions and methods of the present invention include a cationic lipid having a compound structure of: 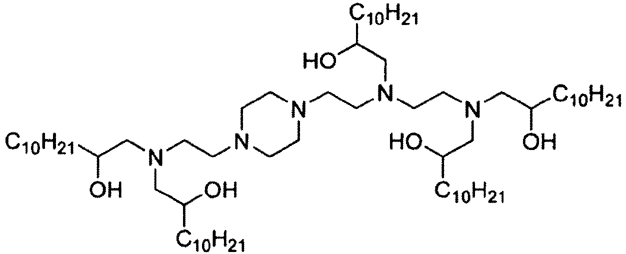 and pharmaceutically acceptable salts thereof.

Other suitable cationic lipids for use in the compositions and methods of the invention include the cationic lipids as described in International Patent Publication WO 2016/118725. In certain embodiments, the compositions and methods of the present invention include a cationic lipid having a compound structure of: 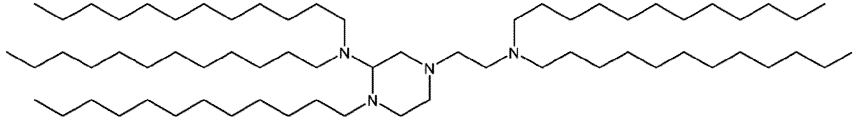 and pharmaceutically acceptable salts thereof.

Other suitable cationic lipids for use in the compositions and methods of the invention include the cationic lipids as described in International Patent Publication WO 2016/118724. In certain embodiments, the compositions and methods of the present invention include a cationic lipid having a compound structure of: 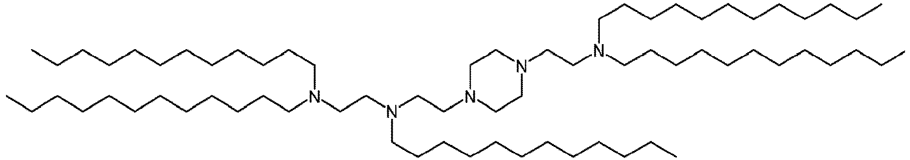 and pharmaceutically acceptable salts thereof.

Other suitable cationic lipids for use in the compositions and methods of the invention include a cationic lipid having the formula of 14,25-ditridecyl 15,18,21,24-tetraaza-octatriacontane, and pharmaceutically acceptable salts thereof.

Other suitable cationic lipids for use in the compositions and methods of the invention include the cationic lipids as described in International Patent Publications WO 2013/063468 and WO 2016/205691. In some embodiments, the compositions and methods of the present invention include a cationic lipid of the following formula: 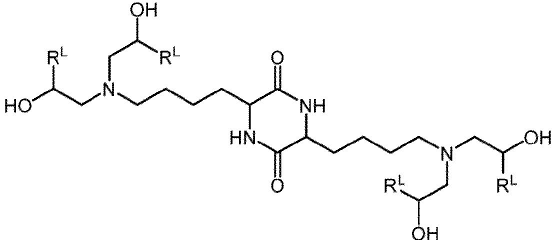 or pharmaceutically acceptable salts thereof, wherein each instance of R^{L} is independently optionally substituted C₆-C₄₀ alkenyl. In certain embodiments, the compositions and methods of the present invention include a cationic lipid having a compound structure of: 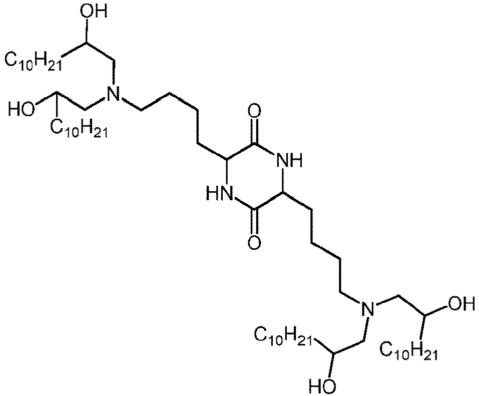 and pharmaceutically acceptable salts thereof. In certain embodiments, the compositions and methods of the present invention include a cationic lipid having a compound structure of: 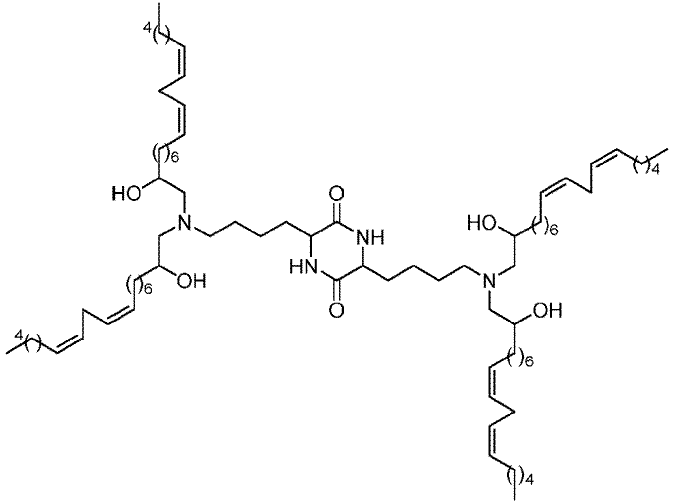 and pharmaceutically acceptable salts thereof. In certain embodiments, the compositions and methods of the present invention include a cationic lipid having a compound structure of: 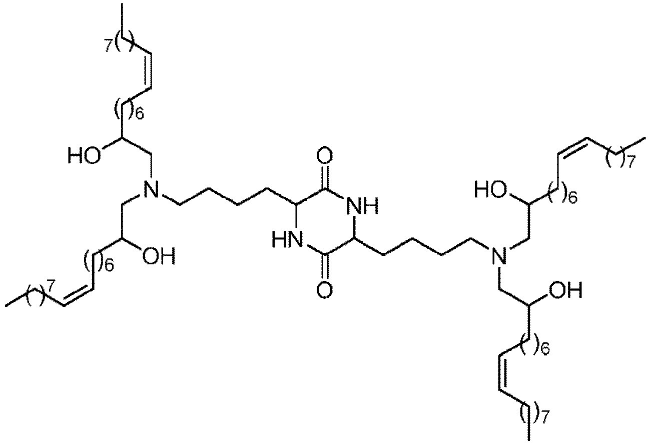 and pharmaceutically acceptable salts thereof. In certain embodiments, the compositions and methods of the present invention include a cationic lipid having a compound structure of: 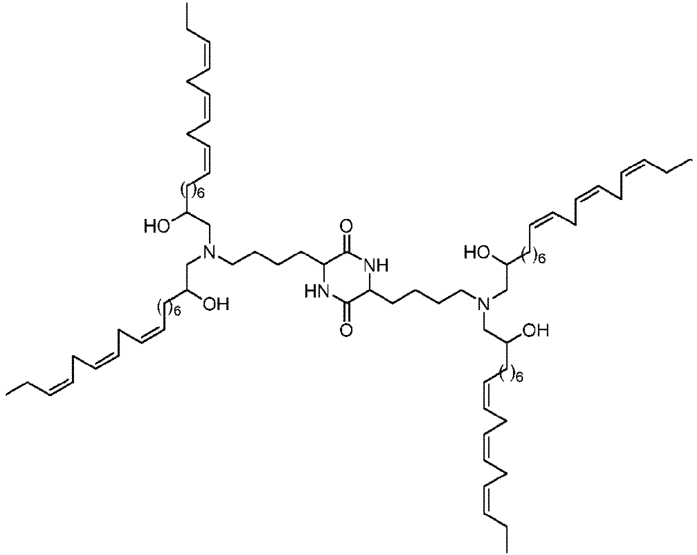 and pharmaceutically acceptable salts thereof.

Other suitable cationic lipids for use in the compositions and methods of the invention include the cationic lipids as described in International Patent Publication WO 2015/184256. In some embodiments, the compositions and methods of the present invention include a cationic lipid of the following formula: 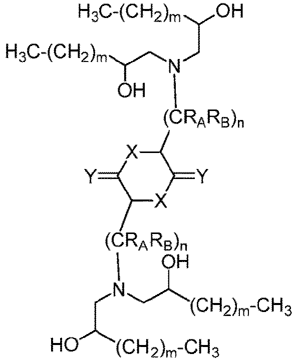 or a pharmaceutically acceptable salt thereof, wherein each X independently is O or S; each Y independently is O or S; each m independently is 0 to 20; each n independently is 1 to 6; each R_{A} is independently hydrogen, optionally substituted C1-50 alkyl, optionally substituted C2-50 alkenyl, optionally substituted C2-50 alkynyl, optionally substituted C3-10 carbocyclyl, optionally substituted 3-14 membered heterocyclyl, optionally substituted C6-14 aryl, optionally substituted 5-14 membered heteroaryl or halogen; and each R_{B} is independently hydrogen, optionally substituted C1-50 alkyl, optionally substituted C2-50 alkenyl, optionally substituted C2-50 alkynyl, optionally substituted C3-10 carbocyclyl, optionally substituted 3-14 membered heterocyclyl, optionally substituted C6-14 aryl, optionally substituted 5-14 membered heteroaryl or halogen. **In** certain embodiments, the compositions and methods of the present invention include a cationic lipid, "Target 23", having a compound structure of: 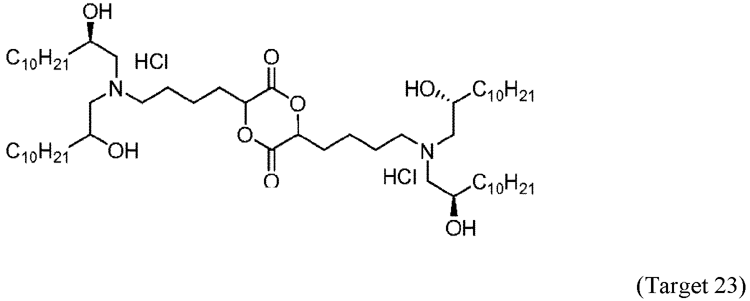 and pharmaceutically acceptable salts thereof.

Other suitable cationic lipids for use in the compositions and methods of the invention include the cationic lipids as described in International Patent Publication WO 2016/004202. In some embodiments, the compositions and methods of the present invention include a cationic lipid having the compound structure: 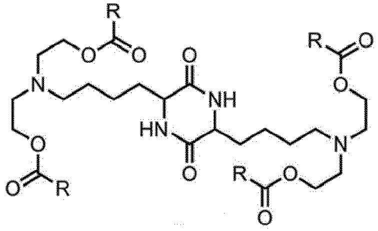 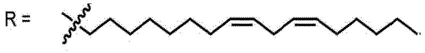 or a pharmaceutically acceptable salt thereof. In some embodiments, the compositions and methods of the present invention include a cationic lipid having the compound structure: 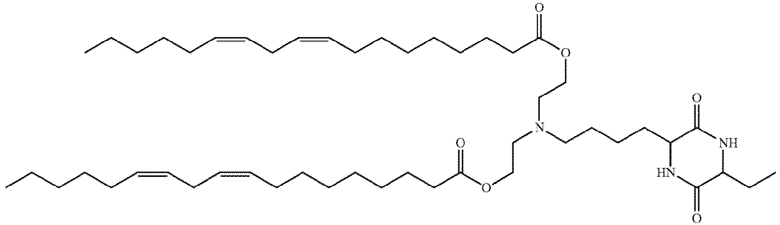 or a pharmaceutically acceptable salt thereof. In some embodiments, the compositions and methods of the present invention include a cationic lipid having the compound structure: 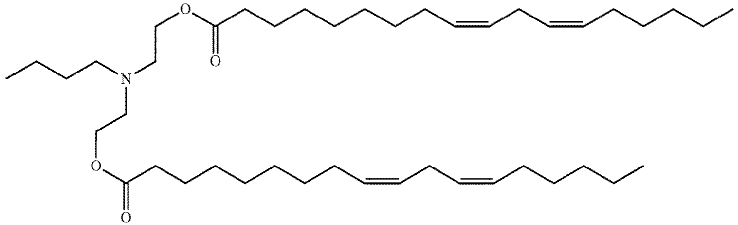 or a pharmaceutically acceptable salt thereof.

Other suitable cationic lipids for use in the compositions and methods of the present invention include cationic lipids as described in United States Provisional Patent Application Serial Number 62/758,179. In some embodiments, the compositions and methods of the present invention include a cationic lipid of the following formula: 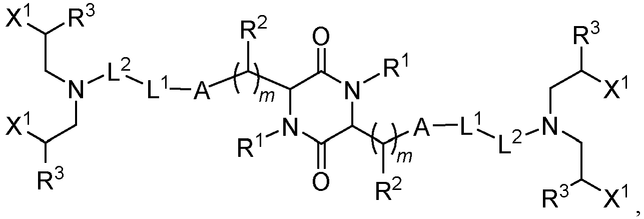 or a pharmaceutically acceptable salt thereof, wherein each R¹ and R² is independently H or C₁-C₆ aliphatic; each *m* is independently an integer having a value of 1 to 4; each A is independently a covalent bond or arylene; each L¹ is independently an ester, thioester, disulfide, or anhydride group; each L₂ is independently C₂-C₁₀ aliphatic; each X¹ is independently H or OH; and each R³ is independently C₆-C₂₀ aliphatic. In some embodiments, the compositions and methods of the present invention include a cationic lipid of the following formula: 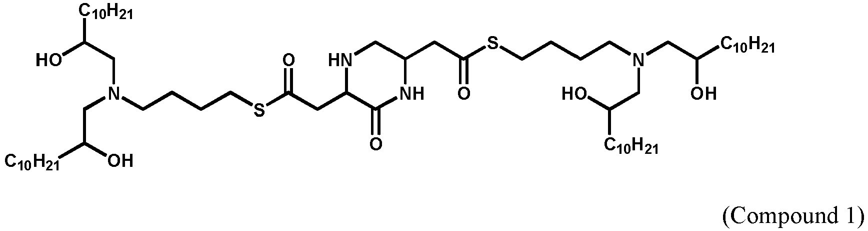 or a pharmaceutically acceptable salt thereof. In some embodiments, the compositions and methods of the present invention include a cationic lipid of the following formula: 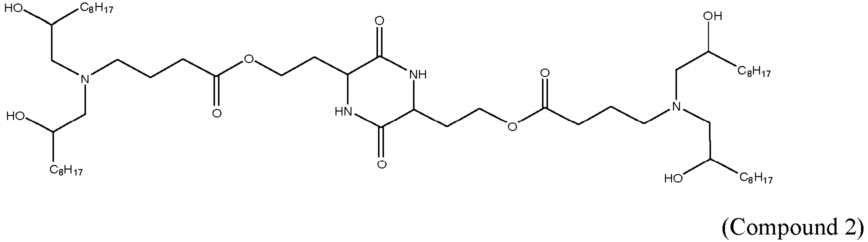 or a pharmaceutically acceptable salt thereof. In some embodiments, the compositions and methods of the present invention include a cationic lipid of the following formula: 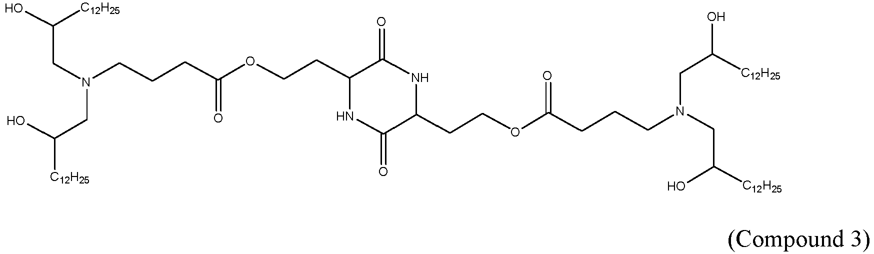 or a pharmaceutically acceptable salt thereof.

Other suitable cationic lipids for use in the compositions and methods of the present invention include the cationic lipids as described in J. McClellan, M. C. King, Cell 2010, 141, 210-217 and in Whitehead et al., Nature Communications (2014) 5:4277. In certain embodiments, the cationic lipids of the compositions and methods of the present invention include a cationic lipid having a compound structure of: 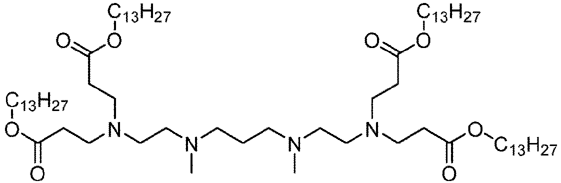 and pharmaceutically acceptable salts thereof.

Other suitable cationic lipids for use in the compositions and methods of the invention include the cationic lipids as described in International Patent Publication WO 2015/199952. In some embodiments, the compositions and methods of the present invention include a cationic lipid having the compound structure: 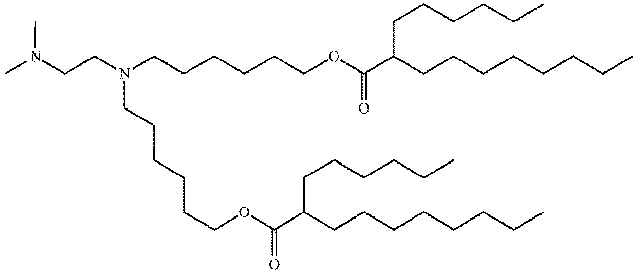 and pharmaceutically acceptable salts thereof. In some embodiments, the compositions and methods of the present invention include a cationic lipid having the compound structure: 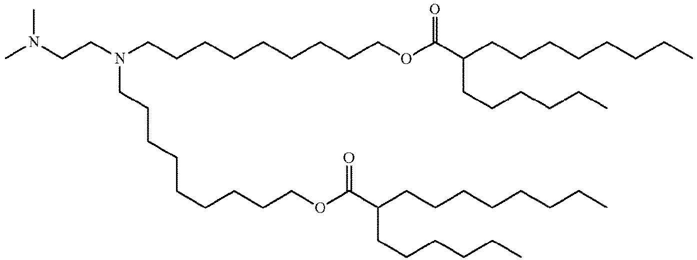 and pharmaceutically acceptable salts thereof. In some embodiments, the compositions and methods of the present invention include a cationic lipid having the compound structure: 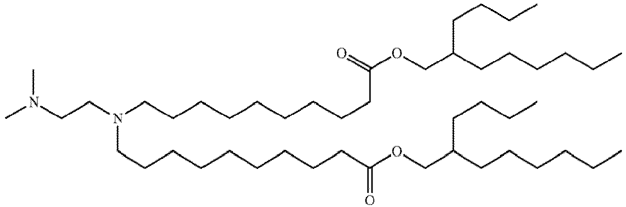 and pharmaceutically acceptable salts thereof. In some embodiments, the compositions and methods of the present invention include a cationic lipid having the compound structure: 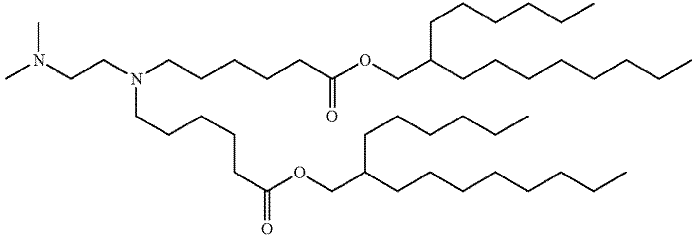 and pharmaceutically acceptable salts thereof. In some embodiments, the compositions and methods of the present invention include a cationic lipid having the compound structure: 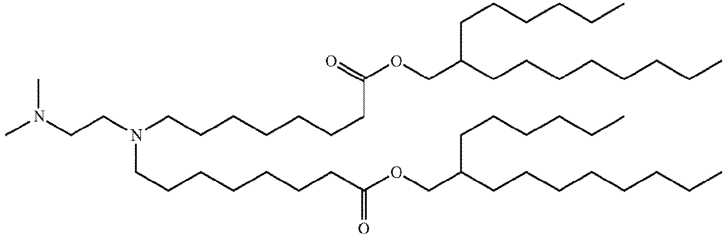 and pharmaceutically acceptable salts thereof. In some embodiments, the compositions and methods of the present invention include a cationic lipid having the compound structure: 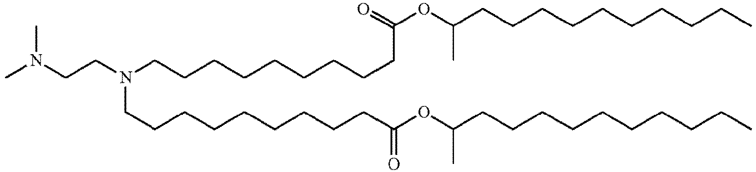 and pharmaceutically acceptable salts thereof. In some embodiments, the compositions and methods of the present invention include a cationic lipid having the compound structure: 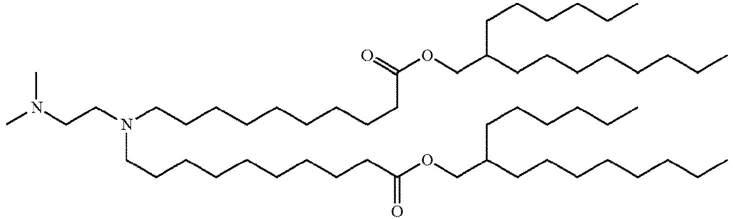 and pharmaceutically acceptable salts thereof. In some embodiments, the compositions and methods of the present invention include a cationic lipid having the compound structure: 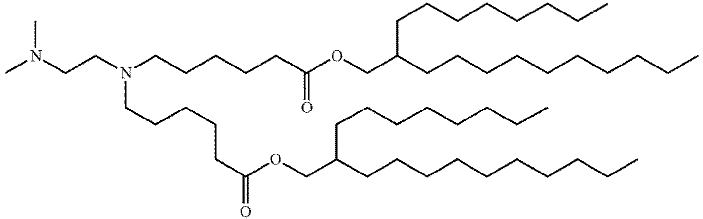 and pharmaceutically acceptable salts thereof. In some embodiments, the compositions and methods of the present invention include a cationic lipid having the compound structure: 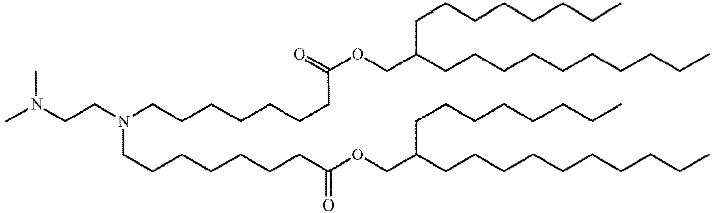 and pharmaceutically acceptable salts thereof. In some embodiments, the compositions and methods of the present invention include a cationic lipid having the compound structure: 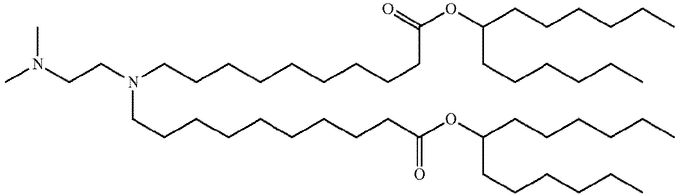 and pharmaceutically acceptable salts thereof. In some embodiments, the compositions and methods of the present invention include a cationic lipid having the compound structure: 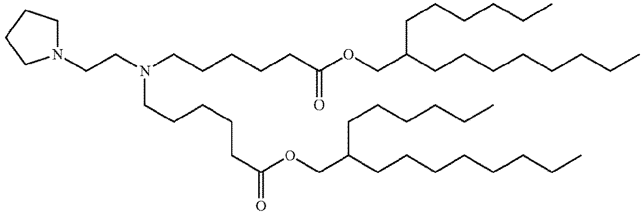 and pharmaceutically acceptable salts thereof. In some embodiments, the compositions and methods of the present invention include a cationic lipid having the compound structure: 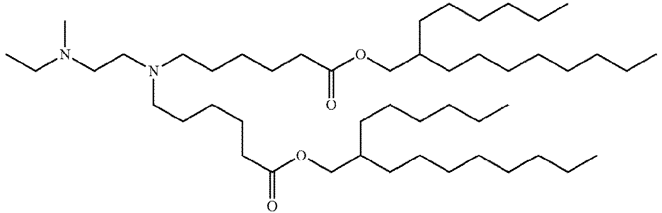 and pharmaceutically acceptable salts thereof. In some embodiments, the compositions and methods of the present invention include a cationic lipid having the compound structure: 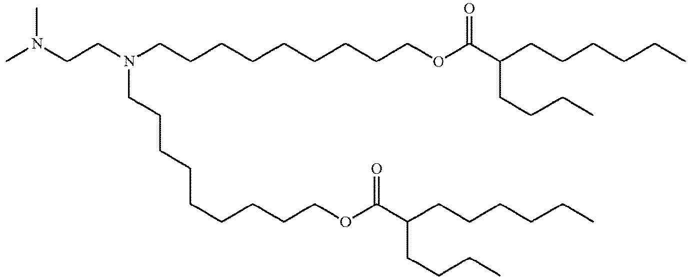 and pharmaceutically acceptable salts thereof.

Other suitable cationic lipids for use in the compositions and methods of the invention include the cationic lipids as described in International Patent Publication WO 2017/004143. In some embodiments, the compositions and methods of the present invention include a cationic lipid having the compound structure: 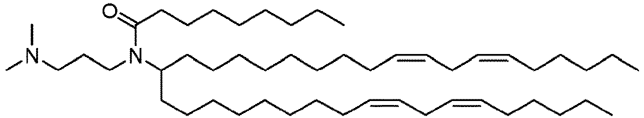 and pharmaceutically acceptable salts thereof. In some embodiments, the compositions and methods of the present invention include a cationic lipid having the compound structure: 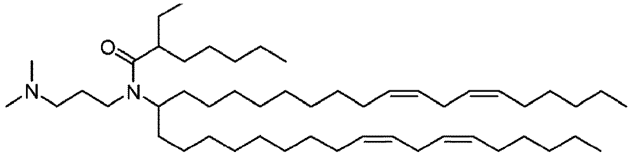 and pharmaceutically acceptable salts thereof. In some embodiments, the compositions and methods of the present invention include a cationic lipid having the compound structure: 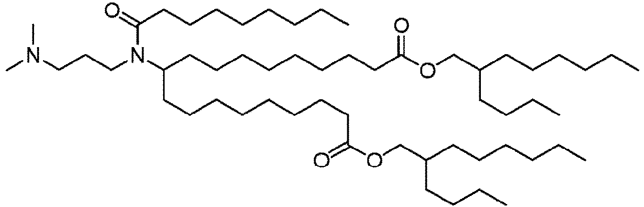 and pharmaceutically acceptable salts thereof. In some embodiments, the compositions and methods of the present invention include a cationic lipid having the compound structure: 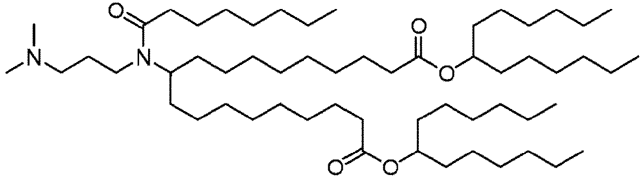 and pharmaceutically acceptable salts thereof. In some embodiments, the compositions and methods of the present invention include a cationic lipid having the compound structure: 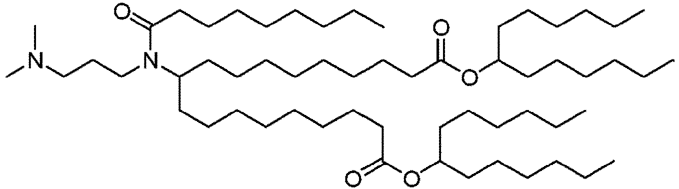 and pharmaceutically acceptable salts thereof. In some embodiments, the compositions and methods of the present invention include a cationic lipid having the compound structure: 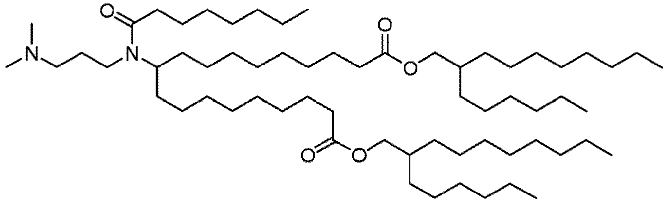 and pharmaceutically acceptable salts thereof. In some embodiments, the compositions and methods of the present invention include a cationic lipid having the compound structure: 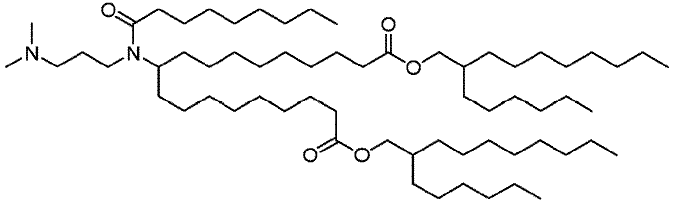 and pharmaceutically acceptable salts thereof. In some embodiments, the compositions and methods of the present invention include a cationic lipid having the compound structure: 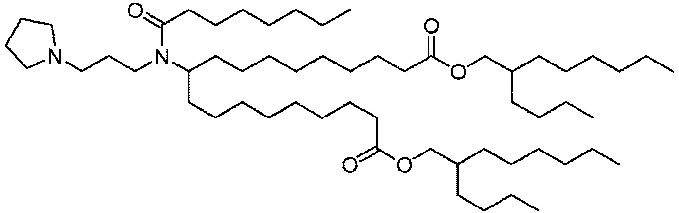 and pharmaceutically acceptable salts thereof. In some embodiments, the compositions and methods of the present invention include a cationic lipid having the compound structure: 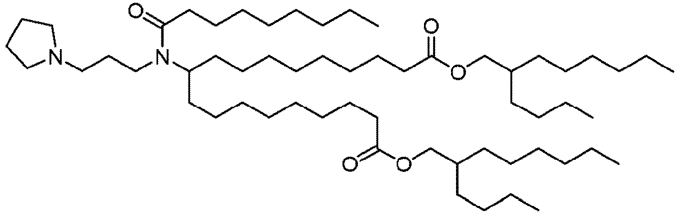 and pharmaceutically acceptable salts thereof. In some embodiments, the compositions and methods of the present invention include a cationic lipid having the compound structure: 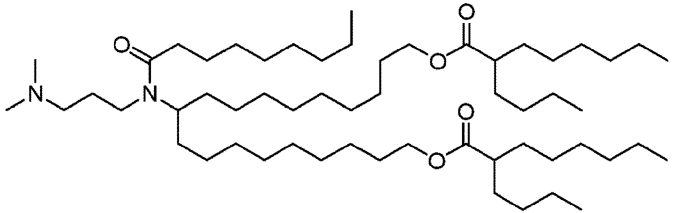 and pharmaceutically acceptable salts thereof. In some embodiments, the compositions and methods of the present invention include a cationic lipid having the compound structure: 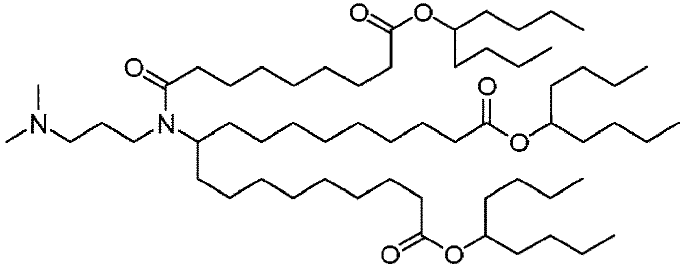 and pharmaceutically acceptable salts thereof. In some embodiments, the compositions and methods of the present invention include a cationic lipid having the compound structure: 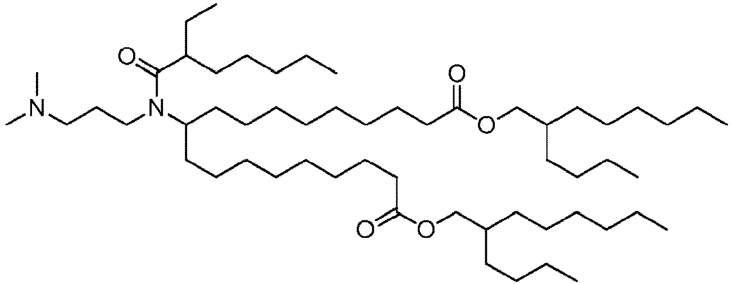 and pharmaceutically acceptable salts thereof. In some embodiments, the compositions and methods of the present invention include a cationic lipid having the compound structure: 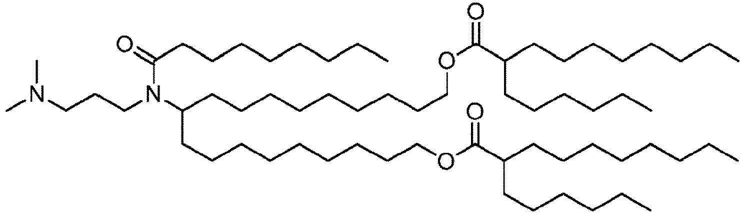 and pharmaceutically acceptable salts thereof. In some embodiments, the compositions and methods of the present invention include a cationic lipid having the compound structure: 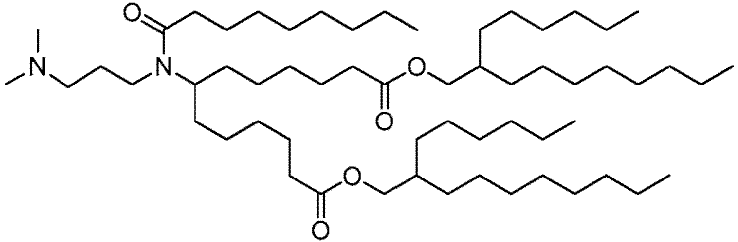 and pharmaceutically acceptable salts thereof. In some embodiments, the compositions and methods of the present invention include a cationic lipid having the compound structure: 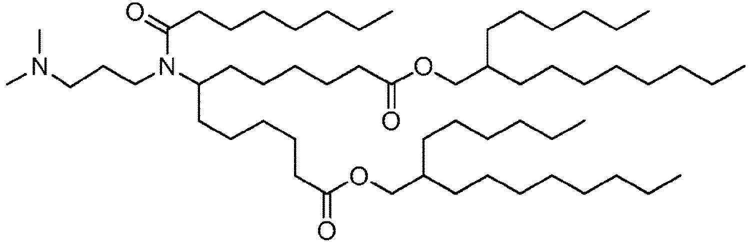 and pharmaceutically acceptable salts thereof. In some embodiments, the compositions and methods of the present invention include a cationic lipid having the compound structure: 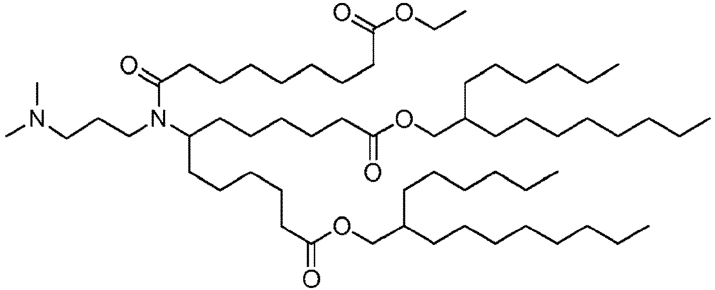 and pharmaceutically acceptable salts thereof. In some embodiments, the compositions and methods of the present invention include a cationic lipid having the compound structure: 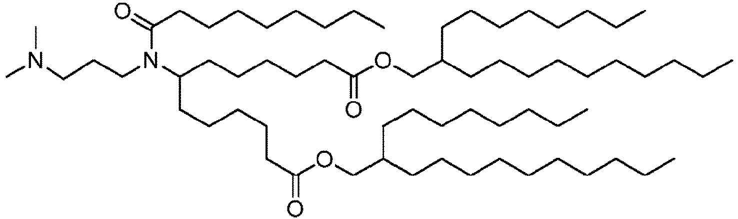 and pharmaceutically acceptable salts thereof.

Other suitable cationic lipids for use in the compositions and methods of the invention include the cationic lipids as described in International Patent Publication WO 2017/075531. In some embodiments, the compositions and methods of the present invention include a cationic lipid of the following formula: 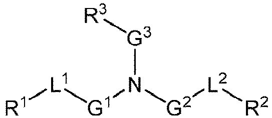 or a pharmaceutically acceptable salt thereof, wherein one of L¹ or L² is -O(C=O)-, -(C=O)O-, - C(=O)-, -O-, -S(O)ₓ, -S-S-, -C(=O)S-, -SC(=O)-, -NR^{a}C(=O)-, -C(=O)NR^{a}-, NR^{a}C(=O)NR^{a}-, - OC(=O)NR^{a}-, or -NR^{a}C(=O)O-; and the other of L¹ or L² is -O(C=O)-, -(C=O)O-, -C(=O)-, -O-, -S(O) ₓ, -S-S-, -C(=O)S-, SC(=O)-, -NR^{a}C(=O)-, -C(=O)NR^{a}-, ,NR^{a}C(=O)NR^{a}-, -OC(=O)NR^{a}- or -NR^{a}C(=O)O- or a direct bond; G¹ and G² are each independently unsubstituted C₁-C₁₂ alkylene or C₁-C₁₂ alkenylene; G³ is C₁-C₂₄ alkylene, C₁-C₂₄ alkenylene, C₃-C₈ cycloalkylene, C₃-C₈ cycloalkenylene; R^{a} is H or C₁-C₁₂ alkyl; R₁ and R₂ are each independently C₆-C₂₄ alkyl or C₆-C₂₄ alkenyl; R³ is H, OR⁵, CN, -C(=O)OR⁴, -OC(=O)R⁴ or -NR⁵ C(=O)R⁴; R⁴ is C₁-C₁₂ alkyl; R⁵ is H or C₁-C₆ alkyl; and x is 0, 1 or 2.

Other suitable cationic lipids for use in the compositions and methods of the invention include the cationic lipids as described in International Patent Publication WO 2017/117528. In some embodiments, the compositions and methods of the present invention include a cationic lipid having the compound structure: 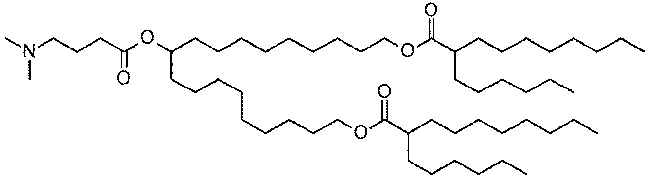 and pharmaceutically acceptable salts thereof. In some embodiments, the compositions and methods of the present invention include a cationic lipid having the compound structure: 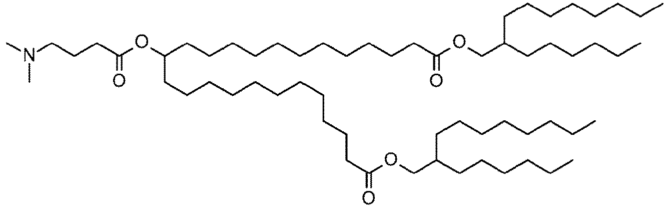 and pharmaceutically acceptable salts thereof. In some embodiments, the compositions and methods of the present invention include a cationic lipid having the compound structure: 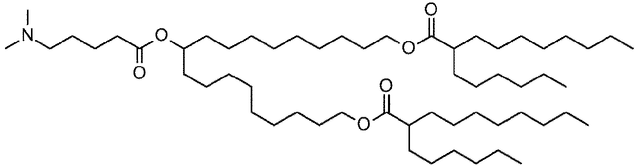 and pharmaceutically acceptable salts thereof.

Other suitable cationic lipids for use in the compositions and methods of the invention include the cationic lipids as described in International Patent Publication WO 2017/049245. In some embodiments, the cationic lipids of the compositions and methods of the present invention include a compound of one of the following formulas: 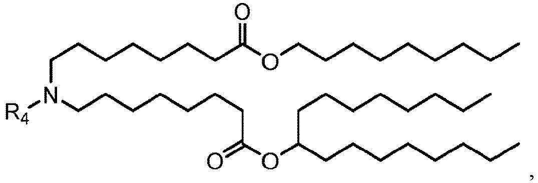 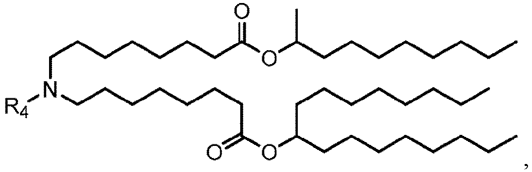 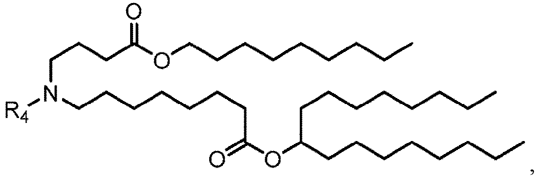 and 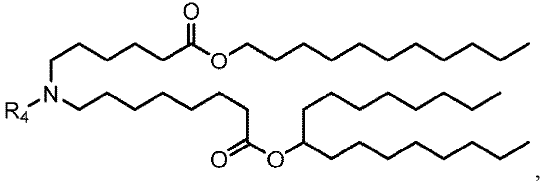 and pharmaceutically acceptable salts thereof. For any one of these four formulas, R₄ is independently selected from -(CH₂)ₙQ and -(CH₂) ₙCHQR; Q is selected from the group consisting of -OR, -OH, -O(CH₂)ₙN(R)₂, -OC(O)R, -CX₃, -CN, -N(R)C(O)R, -N(H)C(O)R, - N(R)S(O)₂R, -N(H)S(O)₂R, -N(R)C(O)N(R)₂, -N(H)C(O)N(R)₂, -N(H)C(O)N(H)(R), - N(R)C(S)N(R)₂, -N(H)C(S)N(R)₂, -N(H)C(S)N(H)(R), and a heterocycle; and n is 1, 2, or 3. In certain embodiments, the compositions and methods of the present invention include a cationic lipid having a compound structure of: 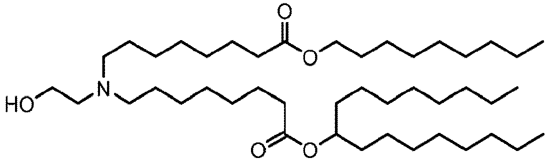 and pharmaceutically acceptable salts thereof. In certain embodiments, the compositions and methods of the present invention include a cationic lipid having a compound structure of: 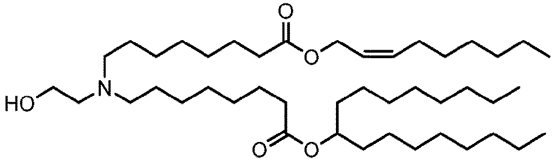 and pharmaceutically acceptable salts thereof. In certain embodiments, the compositions and methods of the present invention include a cationic lipid having a compound structure of: 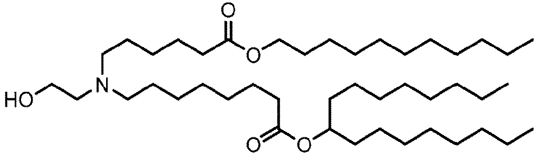 and pharmaceutically acceptable salts thereof. In certain embodiments, the compositions and methods of the present invention include a cationic lipid having a compound structure of: 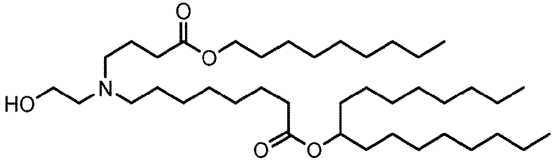 and pharmaceutically acceptable salts thereof.

Other suitable cationic lipids for use in the compositions and methods of the invention include the cationic lipids as described in International Patent Publication WO 2017/173054 and WO 2015/095340. In certain embodiments, the compositions and methods of the present invention include a cationic lipid having a compound structure of: 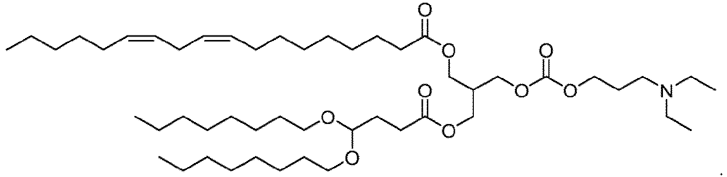 and pharmaceutically acceptable salts thereof. In certain embodiments, the compositions and methods of the present invention include a cationic lipid having a compound structure of: 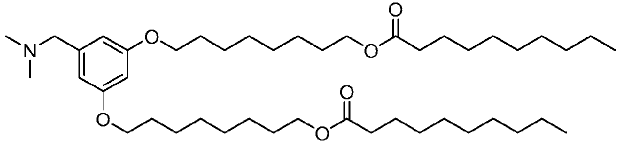 and pharmaceutically acceptable salts thereof. In certain embodiments, the compositions and methods of the present invention include a cationic lipid having a compound structure of: 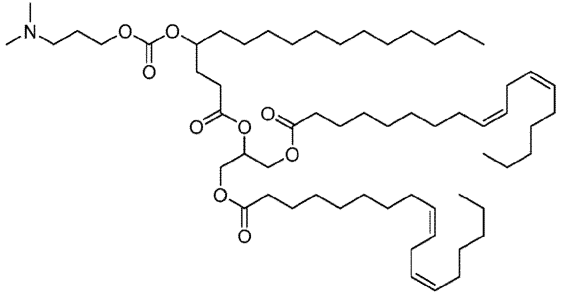 and pharmaceutically acceptable salts thereof. In certain embodiments, the compositions and methods of the present invention include a cationic lipid having a compound structure of: 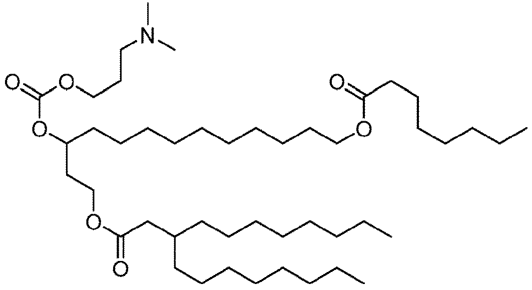 and pharmaceutically acceptable salts thereof.

Other suitable cationic lipids for use in the compositions and methods of the present invention include cleavable cationic lipids as described in International Patent Publication WO 2012/170889. In some embodiments, the compositions and methods of the present invention include a cationic lipid of the following formula: 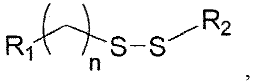 wherein R₁ is selected from the group consisting of imidazole, guanidinium, amino, imine, enamine, an optionally-substituted alkyl amino (e.g., an alkyl amino such as dimethylamino) and pyridyl; wherein R₂ is selected from the group consisting of one of the following two formulas: 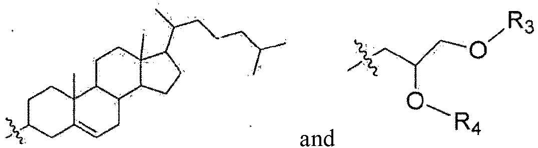 and wherein R₃ and R₄ are each independently selected from the group consisting of an optionally substituted, variably saturated or unsaturated C₆-C₂₀ alkyl and an optionally substituted, variably saturated or unsaturated C₆-C₂₀ acyl; and wherein n is zero or any positive integer (e.g., one, two, three, four, five, six, seven, eight, nine, ten, eleven, twelve, thirteen, fourteen, fifteen, sixteen, seventeen, eighteen, nineteen, twenty or more). In certain embodiments, the compositions and methods of the present invention include a cationic lipid, "HGT4001", having a compound structure of: 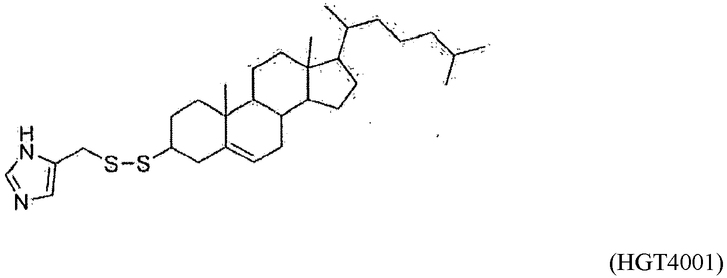 and pharmaceutically acceptable salts thereof. In certain embodiments, the compositions and methods of the present invention include a cationic lipid, "HGT4002", having a compound structure of: 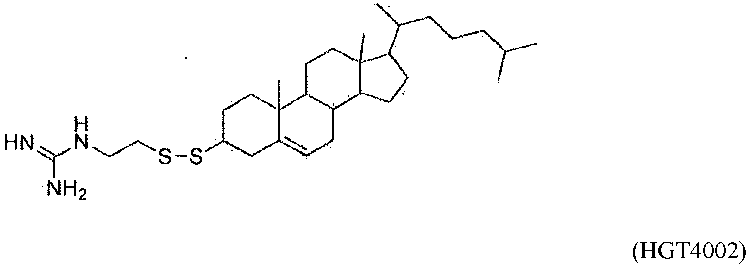 and pharmaceutically acceptable salts thereof. In certain embodiments, the compositions and methods of the present invention include a cationic lipid, "HGT4003", having a compound structure of: 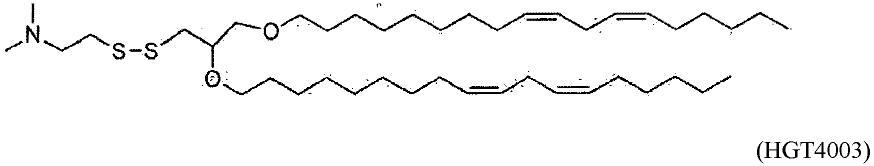 and pharmaceutically acceptable salts thereof. In certain embodiments, the compositions and methods of the present invention include a cationic lipid, "HGT4004", having a compound structure of: 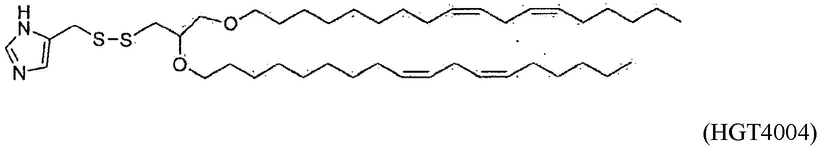 and pharmaceutically acceptable salts thereof. In certain embodiments, the compositions and methods of the present invention include a cationic lipid "HGT4005", having a compound structure of: 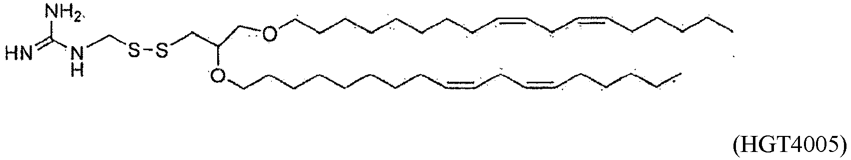 and pharmaceutically acceptable salts thereof.

Other suitable cationic lipids for use in the compositions and methods of the present invention include cleavable cationic lipids as described in International Application No. PCT/US2019/032522. In certain embodiments, the compositions and methods of the present invention include a cationic lipid that is any of general formulas or any of structures (1a)-(21a) and (1b)-(21b) and (22)-(237) described in International Application No. PCT/US2019/032522. In certain embodiments, the compositions and methods of the present invention include a cationic lipid that has a structure according to Formula (I'), 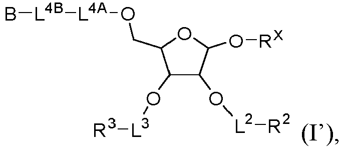 wherein:
R^{X} is independently -H, -L¹-R¹, or -L^{5A}-L^{5B}-B';
each of L¹, L², and L³ is independently a covalent bond, -C(O)-, -C(O)O-, -C(O)S-, or - C(O)NR^{L}-;
each L^{4A} and L^{5A} is independently -C(O)-, -C(O)O-, or -C(O)NR^{L}-;
each L^{4B} and L^{5B} is independently C₁-C₂₀ alkylene; C₂-C₂₀ alkenylene; or C₂-C₂₀ alkynylene;
each B and B' is NR⁴R⁵ or a 5- to 10-membered nitrogen-containing heteroaryl;
each R¹, R², and R³ is independently C₆-C₃₀ alkyl, C₆-C₃₀ alkenyl, or C₆-C₃₀ alkynyl;
each R⁴ and R⁵ is independently hydrogen, C₁-C₁₀ alkyl; C₂-C₁₀ alkenyl; or C₂-C₁₀ alkynyl; and
each R^{L} is independently hydrogen, C₁-C₂₀ alkyl, C₂-C₂₀ alkenyl, or C₂-C₂₀ alkynyl.

In certain embodiments, the compositions and methods of the present invention include a cationic lipid that is Compound (139) of 62/672,194, having a compound structure of:

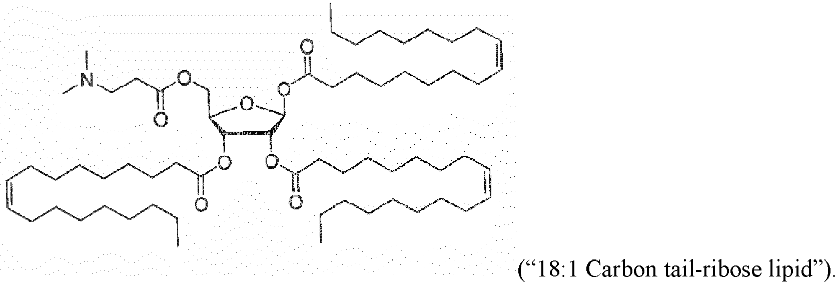

In some embodiments, the compositions and methods of the present invention include the cationic lipid, N-[1-(2,3-dioleyloxy)propyl]-N,N,N-trimethylammonium chloride ("DOTMA"). (Feigner et al. (Proc. Nat'l Acad. Sci. 84, 7413 (1987); U.S. Pat. No. 4,897,355). Other cationic lipids suitable for the compositions and methods of the present invention include, for example, 5-carboxyspermylglycinedioctadecylamide ("DOGS"); 2,3-dioleyloxy-N-[2(spermine-carboxamido)ethyl]-N,N-dimethyl-1-propanaminium ("DOSPA") (Behr et al. Proc. Nat.'l Acad. Sci. 86, 6982 (1989), U.S. Pat. No. 5,171,678; U.S. Pat. No. 5,334,761); 1,2-Dioleoyl-3-Dimethylammonium-Propane ("DODAP"); 1,2-Dioleoyl-3-Trimethylammonium-Propane ("DOTAP").

Additional exemplary cationic lipids suitable for the compositions and methods of the present invention also include: 1,2-distearyloxy-N,N-dimethyl-3-aminopropane ( "DSDMA"); 1,2-dioleyloxy-N,N-dimethyl-3-aminopropane ("DODMA"); 1 ,2-dilinoleyloxy-N,N-dimethyl-3-aminopropane ("DLinDMA"); 1,2-dilinolenyloxy-N,N-dimethyl-3-aminopropane ("DLenDMA"); N-dioleyl-N,N-dimethylammonium chloride ("DODAC"); N,N-distearyl-N,N-dimethylammonium bromide ("DDAB"); N-(1,2-dimyristyloxyprop-3-yl)-N,N-dimethyl-N-hydroxyethyl ammonium bromide ("DMRIE"); 3-dimethylamino-2-(cholest-5-en-3-beta-oxybutan-4-oxy)-1-(cis,cis-9,12-octadecadienoxy)propane ("CLinDMA"); 2-[5'-(cholest-5-en-3-beta-oxy)-3'-oxapentoxy)-3-dimethy 1-1-(cis,cis-9', 1-2'-octadecadienoxy)propane ("CpLinDMA"); N,N-dimethyl-3,4-dioleyloxybenzylamine ("DMOBA"); 1 ,2-N,N'-dioleylcarbamyl-3-dimethylaminopropane ("DOcarbDAP"); 2,3-Dilinoleoyloxy-N,N-dimethylpropylamine ("DLinDAP"); 1,2-N,N'-Dilinoleylcarbamyl-3-dimethylaminopropane ("DLincarbDAP"); 1,2-Dilinoleoylcarbamyl-3-dimethylaminopropane ("DLinCDAP"); 2,2-dilinoleyl-4-dimethylaminomethyl-[1,3]-dioxolane ("DLin-K-DMA"); 2-((8-[(3P)-cholest-5-en-3-yloxy]octyl)oxy)-N, N-dimethyl-3-[(9Z, 12Z)-octadeca-9, 12-dien-1 -yloxy]propane-1-amine ("Octyl-CLinDMA"); (2R)-2-((8-[(3beta)-cholest-5-en-3-yloxy]octyl)oxy)-N, N-dimethyl-3-[(9Z, 12Z)-octadeca-9, 12-dien-1-yloxy]propan-1 -amine ("Octyl-CLinDMA (2R)"); (2S)-2-((8-[(3P)-cholest-5-en-3-yloxy]octyl)oxy)-N, fsl-dimethyh3-[(9Z, 12Z)-octadeca-9, 12-dien-1 - yloxy]propan-1 -amine ("Octyl-CLinDMA (2S)"); 2,2-dilinoleyl-4-dimethylaminoethyl-[1,3]-dioxolane ("DLin-K-XTC2-DMA"); and 2-(2,2-di((9Z,12Z)-octadeca-9,12-dien- 1-yl)-1,3-dioxolan-4-yl)-N,N-dimethylethanamine ("DLin-KC2-DMA") (see, WO 2010/042877; Semple et al., Nature Biotech. 28: 172-176 (2010)). (Heyes, J., et al., J Controlled Release 107: 276-287 (2005); Morrissey, DV. et al., Nat. Biotechnol. 23(8): 1003-1007 (2005); International Patent Publication WO 2005/121348). In some embodiments, one or more of the cationic lipids comprise at least one of an imidazole, dialkylamino, or guanidinium moiety.

In some embodiments, one or more cationic lipids suitable for the compositions and methods of the present invention include 2,2-Dilinoleyl-4-dimethylaminoethyl-[1,3]-dioxolane ("XTC"); (3aR,5s,6aS)-N,N-dimethyl-2,2-di((9Z,12Z)-octadeca-9,12-dienyl)tetrahydro-3aH-cyclopenta[d] [1 ,3]dioxol-5-amine ("ALNY-100") and/or 4,7,13-tris(3-oxo-3-(undecylamino)propyl)-N1,N16-diundecyl-4,7,10,13-tetraazahexadecane-1,16-diamide ("NC98-5").

In some embodiments, the compositions of the present invention include one or more cationic lipids that constitute at least about 5%, 10%, 20%, 30%, 35%, 40%, 45%, 50%, 55%, 60%, 65%, or 70%, measured by weight, of the total lipid content in the composition, e.g., a lipid nanoparticle. In some embodiments, the compositions of the present invention include one or more cationic lipids that constitute at least about 5%, 10%, 20%, 30%, 35%, 40%, 45%, 50%, 55%, 60%, 65%, or 70%, measured as a mol %, of the total lipid content in the composition, e.g., a lipid nanoparticle. In some embodiments, the compositions of the present invention include one or more cationic lipids that constitute about 30-70 % (e.g., about 30-65%, about 30-60%, about 30-55%, about 30-50%, about 30-45%, about 30-40%, about 35-50%, about 35-45%, or about 35-40%), measured by weight, of the total lipid content in the composition, e.g., a lipid nanoparticle. In some embodiments, the compositions of the present invention include one or more cationic lipids that constitute about 30-70 % (e.g., about 30-65%, about 30-60%, about 30-55%, about 30-50%, about 30-45%, about 30-40%, about 35-50%, about 35-45%, or about 35-40%), measured as mol %, of the total lipid content in the composition, e.g., a lipid nanoparticle

In some embodiments, sterol-based cationic lipids may be use instead or in addition to cationic lipids described herein. Suitable sterol-based cationic lipids are dialkylamino-, imidazole-, and guanidinium-containing sterol-based cationic lipids. For example, certain embodiments are directed to a composition comprising one or more sterol-based cationic lipids comprising an imidazole, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate, as represented by structure (I) below. In certain embodiments, a lipid nanoparticle for delivery of RNA (e.g., mRNA) encoding a functional protein may comprise one or more imidazole-based cationic lipids, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate, as represented by the following structure: 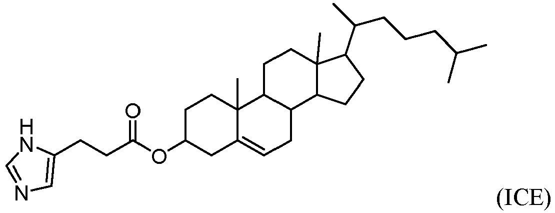 In some embodiments, the percentage of cationic lipid in a liposome may be greater than 10%, greater than 20%, greater than 30%, greater than 40%, greater than 50%, greater than 60%, or greater than 70%. In some embodiments, cationic lipid(s) constitute(s) about 30-50 % (e.g., about 30-45%, about 30-40%, about 35-50%, about 35-45%, or about 35-40%) of the liposome by weight. In some embodiments, the cationic lipid (e.g., ICE lipid) constitutes about 30%, about 35%, about 40 %, about 45%, or about 50% of the liposome by molar ratio.

### Non-Cationic/Helper Lipids

In some embodiments, the liposomes contain one or more non-cationic ("helper") lipids. As used herein, the phrase "non-cationic lipid" refers to any neutral, zwitterionic or anionic lipid. As used herein, the phrase "anionic lipid" refers to any of a number of lipid species that carry a net negative charge at a selected H, such as physiological pH. Non-cationic lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoyl-phosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidylethanolamine (DSPE), 1,2-dierucoyl-sn-glycero-3-phosphoethanolamine (DEPE), phosphatidylserine, sphingolipids, cerebrosides, gangliosides, 16-O-monomethyl PE, 16-O-dimethyl PE, 18-1-trans PE, l-stearoyl-2-oleoyl-phosphatidyethanolamine (SOPE), or a mixture thereof. In some embodiments, liposomes suitable for use with the invention include DOPE as the non-cationic lipid component. In other embodiments, liposomes suitable for use with the invention include DEPE as the non-cationic lipid component.

In some embodiments, a non-cationic lipid is a neutral lipid, *i.e.,* a lipid that does not carry a net charge in the conditions under which the composition is formulated and/or administered.

In some embodiments, a non-cationic lipid may be present in a molar ratio (mol%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, total non-cationic lipids may be present in a molar ratio (mol%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, the percentage of non-cationic lipid in a liposome may be greater than about 5 mol%, greater than about 10 mol%, greater than about 20 mol%, greater than about 30 mol%, or greater than about 40 mol%. In some embodiments, the percentage total non-cationic lipids in a liposome may be greater than about 5 mol%, greater than about 10 mol%, greater than about 20 mol%, greater than about 30 mol%, or greater than about 40 mol%. In some embodiments, the percentage of non-cationic lipid in a liposome is no more than about 5 mol%, no more than about 10 mol%, no more than about 20 mol%, no more than about 30 mol%, or no more than about 40 mol%. In some embodiments, the percentage total non-cationic lipids in a liposome may be no more than about 5 mol%, no more than about 10 mol%, no more than about 20 mol%, no more than about 30 mol%, or no more than about 40 mol%.

In some embodiments, a non-cationic lipid may be present in a weight ratio (wt%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, total non-cationic lipids may be present in a weight ratio (wt%) of about 5% to about 90%, about 5% to about 70%, about 5% to about 50%, about 5% to about 40%, about 5% to about 30%, about 10 % to about 70%, about 10% to about 50%, or about 10% to about 40% of the total lipids present in a composition. In some embodiments, the percentage of non-cationic lipid in a liposome may be greater than about 5 wt%, greater than about 10 wt%, greater than about 20 wt%, greater than about 30 wt%, or greater than about 40 wt%. In some embodiments, the percentage total non-cationic lipids in a liposome may be greater than about 5 wt%, greater than about 10 wt%, greater than about 20 wt%, greater than about 30 wt%, or greater than about 40 wt%. In some embodiments, the percentage of non-cationic lipid in a liposome is no more than about 5 wt%, no more than about 10 wt%, no more than about 20 wt%, no more than about 30 wt%, or no more than about 40 wt%. In some embodiments, the percentage total non-cationic lipids in a liposome may be no more than about 5 wt%, no more than about 10 wt%, no more than about 20 wt%, no more than about 30 wt%, or no more than about 40 wt%.

### PEGylated Lipids

In some embodiments, a suitable lipid solution includes one or more PEGylated lipids.

For example, the use of polyethylene glycol (PEG)-modified phospholipids and derivatized lipids such as derivatized ceramides (PEG-CER), including N-Octanoyl-Sphingosine-l-[Succinyl(Methoxy Polyethylene Glycol)-2000] (C8 PEG-2000 ceramide) is also contemplated by the present invention.

Contemplated PEG-modified lipids include, but are not limited to, a polyethylene glycol chain of up to 2kDa, up to 3 kDa, up to 4kDa or up to 5 kDa in length covalently attached to a lipid with alkyl chain(s) of C₆-C₂₀ length. In some embodiments, a PEG-modified or PEGylated lipid is PEGylated cholesterol or PEG-2K. In some embodiments, particularly useful exchangeable lipids are PEG-ceramides having shorter acyl chains (e.g., C₁₄ or C₁₈). The addition of such components may prevent complex aggregation and may also provide a means for increasing circulation lifetime and increasing the delivery of the lipid-nucleic acid composition to the target tissues, (Klibanov et al. (1990) FEBS Letters, 268 (1): 235-237), or they may be selected to rapidly exchange out of the formulation *in vivo* (see U.S. Pat. No. 5,885,613). Particularly useful exchangeable lipids are PEG-ceramides having shorter acyl chains (e.g., C14 or C18). Liposomes suitable for use with the invention typically include a PEG-modified lipid such as 1,2-dimyristoyl-rac-glycero-3-methoxypolyethylene glycol-2000 (DMG-PEG2K).

The PEG-modified phospholipid and derivitized lipids may comprise a molar ratio from about 0% to about 20%, about 0.5% to about 20%, about 1% to about 15%, about 4% to about 10%, or about 2% of the total lipid present in the liposomal transfer vehicle.

PEG-modified phospholipid and derivatized lipids may constitute no greater than about 0.5%, 1%, 1.5%, 2%, 2.5%, 3%, 3.5%, 4%, 4.5% or 5% of the total lipids in a suitable lipid solution by weight or by molar. In some embodiments, PEG-modified lipids may constitute about 5% or less of the total lipids in a suitable lipid solution by weight or by molar concentration. In some embodiments, PEG-modified lipids may constitute about 4% or less of the total lipids in a suitable lipid solution by weight or by molar concentration. In some embodiments, PEG-modified lipids typically constitute 3% or less of total lipids in a suitable lipid solution by weight or by molar concentration. In some embodiments, PEG-modified lipids typically constitute 2% or less of total lipids in a suitable lipid solution by weight or by molar concentration. In some embodiments, PEG-modified lipids typically constitute 1% or less of total lipids in a suitable lipid solution by weight or by molar concentration. In some embodiments, PEG-modified lipids constitute about 1-5%, about 1-4%, about 1-3%, or about 1-2%, ) of the total lipids in a suitable lipid solution by weight or by molar concentration. In some embodiments, PEG modified lipids constitute about 0.01-3% (e.g., about 0.01-2.5%, 0.01-2%, 0.01-1.5%, 0.01-1%) of the total lipids in a suitable lipid solution by weight or by molar concentration.

According to various embodiments, the selection of cationic lipids, non-cationic lipids and/or PEG-modified lipids which comprise the lipid nanoparticle, as well as the relative molar ratio of such lipids to each other, is based upon the characteristics of the selected lipid(s), the nature of the intended target cells, the characteristics of the mRNA to be delivered. Additional considerations include, for example, the saturation of the alkyl chain, as well as the size, charge, pH, pKa, fusogenicity and toxicity of the selected lipid(s). Thus the molar ratios may be adjusted accordingly.

Various combinations of lipids, i.e., cationic lipids, non-cationic lipids, PEG-modified lipids and optionally cholesterol, that can used to prepare, and that are comprised in, preformed lipid nanoparticles are described in the literature and herein. For example, a suitable lipid solution may contain cKK-E12, DOPE, cholesterol, and DMG-PEG2K; C12-200, DOPE, cholesterol, and DMG-PEG2K; HGT5000, DOPE, cholesterol, and DMG-PEG2K; HGT5001, DOPE, cholesterol, and DMG-PEG2K; cKK-E12, DPPC, cholesterol, and DMG-PEG2K; C12-200, DPPC, cholesterol, and DMG-PEG2K; HGT5000, DPPC, chol, and DMG-PEG2K; HGT5001, DPPC, cholesterol, and DMG-PEG2K; or ICE, DOPE and DMG-PEG2K. Additional combinations of lipids are described in the art, e.g., PCT/US17/61100, filed on November 10, 2017, published as WO 2018/089790; entitled "Novel ICE-based Lipid Nanoparticle Formulation for Delivery of mRNA,"; PCT/US18/21292, filed on March 7, 2018, published as WO 2018/165257, entitled "PolyAnionic Delivery of Nucleic Acids"; PCT/US18/36920, filed on June 11, 2018, entitled, "Poly (Phosphoesters) for Delivery of Nucleic Acids"; the disclosures of which are included here in their full scope by reference.

In various embodiments, cationic lipids (e.g., cKK-E12, C12-200, ICE, and/or HGT4003) constitute about 30-60 % (e.g., about 30-55%, about 30-50%, about 30-45%, about 30-40%, about 35-50%, about 35-45%, or about 35-40%) of the liposome by molar ratio. In some embodiments, the percentage of cationic lipids (e.g., cKK-E12, C12-200, ICE, and/or HGT4003) is or greater than about 30%, about 35%, about 40 %, about 45%, about 50%, about 55%, or about 60% of the liposome by molar ratio.

In some embodiments, the ratio of cationic lipid(s) to non-cationic lipid(s) to cholesterol-based lipid(s) to PEG-modified lipid(s) may be between about 30-60:25-35:20-30:1-15, respectively. In some embodiments, the ratio of cationic lipid(s) to non-cationic lipid(s) to cholesterol-based lipid(s) to PEG-modified lipid(s) is approximately 40:30:20:10, respectively. In some embodiments, the ratio of cationic lipid(s) to non-cationic lipid(s) to cholesterol-based lipid(s) to PEG-modified lipid(s) is approximately 40:30:25:5, respectively. In some embodiments, the ratio of cationic lipid(s) to non-cationic lipid(s) to cholesterol-based lipid(s) to PEG-modified lipid(s) is approximately 40:32:25:3, respectively. In some embodiments, the ratio of cationic lipid(s) to non-cationic lipid(s) to cholesterol-based lipid(s) to PEG-modified lipid(s) is approximately 50:25:20:5. In some embodiments, the ratio of sterol lipid(s) to non-cationic lipid(s) to PEG-modified lipid(s) is 50:45:5. In some embodiments, the ratio of sterol lipid(s) to non-cationic lipid(s) to PEG-modified lipid(s) is 50:40:10. In some embodiments, the ratio of sterol lipid(s) to non-cationic lipid(s) to PEG-modified lipid(s) is 55:40:5. In some embodiments, the ratio of sterol lipid(s) to non-cationic lipid(s) to PEG-modified lipid(s) is 55:35:10. In some embodiments, the ratio of sterol lipid(s) to non-cationic lipid(s) to PEG-modified lipid(s) is 60:35:5. In some embodiments, the ratio of sterol lipid(s) to non-cationic lipid(s) to PEG-modified lipid(s) is 60:30:10.

In some embodiments, a suitable liposome for the present invention comprises ICE and DOPE at an ICE:DOPE molar ratio of >1:1. In some embodiments, the ICE:DOPE molar ratio is <2.5:1. In some embodiments, the ICE:DOPE molar ratio is between 1:1 and 2.5:1. In some embodiments, the ICE:DOPE molar ratio is approximately 1.5:1. In some embodiments, the ICE:DOPE molar ratio is approximately 1.7:1. In some embodiments, the ICE:DOPE molar ratio is approximately 2:1. In some embodiments, a suitable liposome for the present invention comprises ICE and DMG-PEG-2K at an ICE:DMG-PEG-2K molar ratio of >10:1. In some embodiments, the ICE:DMG-PEG-2K molar ratio is <16:1. In some embodiments, the ICE:DMG-PEG-2K molar ratio is approximately 12:1. In some embodiments, the ICE:DMG-PEG-2K molar ratio is approximately 14:1. In some embodiments, a suitable liposome for the present invention comprises DOPE and DMG-PEG-2K at a DOPE: DMG-PEG-2K molar ratio of >5:1. In some embodiments, the DOPE: DMG-PEG-2K molar ratio is <11:1. In some embodiments, the DOPE: DMG-PEG-2K molar ratio is approximately 7:1. In some embodiments, the DOPE: DMG-PEG-2K molar ratio is approximately 10:1. In some embodiments, a suitable liposome for the present invention comprises ICE, DOPE and DMG-PEG-2K at an ICE:DOPE:DMG-PEG-2K molar ratio of 50:45:5. In some embodiments, a suitable liposome for the present invention comprises ICE, DOPE and DMG-PEG-2K at an ICE:DOPE:DMG-PEG-2K molar ratio of 50:40: 10. In some embodiments, a suitable liposome for the present invention comprises ICE, DOPE and DMG-PEG-2K at an ICE:DOPE:DMG-PEG-2K molar ratio of 55:40:5. In some embodiments, a suitable liposome for the present invention comprises ICE, DOPE and DMG-PEG-2K at an ICE:DOPE:DMG-PEG-2K molar ratio of 55:35:10. In some embodiments, a suitable liposome for the present invention comprises ICE, DOPE and DMG-PEG-2K at an ICE:DOPE:DMG-PEG-2K molar ratio of 60:35:5. In some embodiments, a suitable liposome for the present invention comprises ICE, DOPE and DMG-PEG-2K at an ICE:DOPE:DMG-PEG-2K molar ratio of 60:30:10.

In some embodiments, a suitable delivery vehicle is formulated using a polymer as a carrier, alone or in combination with other carriers including various lipids described herein. Thus, in some embodiments, liposomal delivery vehicles, as used herein, also encompass nanoparticles comprising polymers. Suitable polymers may include, for example, polyacrylates, polyalkycyanoacrylates, polylactide, polylactide-polyglycolide copolymers, polycaprolactones, dextran, albumin, gelatin, alginate, collagen, chitosan, cyclodextrins, protamine, PEGylated protamine, PLL, PEGylated PLL and polyethylenimine (PEI). When PEI is present, it may be branched PEI of a molecular weight ranging from 10 to 40 kDa, e.g., 25 kDa branched PEI (Sigma #408727).

### Formation of Liposomes

The liposomal transfer vehicles for use in the present invention can be prepared by various techniques which are presently known in the art. For example, multilamellar vesicles (MLV) may be prepared according to conventional techniques, such as by depositing a selected lipid on the inside wall of a suitable container or vessel by dissolving the lipid in an appropriate solvent, and then evaporating the solvent to leave a thin film on the inside of the vessel or by spray drying. An aqueous phase may then added to the vessel with a vortexing motion which results in the formation of MLVs. Unilamellar vesicles (ULV) can then be formed by homogenization, sonication or extrusion of the multi-lamellar vesicles. In addition, unilamellar vesicles can be formed by detergent removal techniques.

Various methods are described in published U.S. Application No. US 2011/0244026 , published U.S. Application No. US 2016/0038432 , published U.S. Application No. US 2018/0153822 , published U.S. Application No. US 2018/0125989 and U.S. Provisional Application No. 62/877,597 , filed July 23, 2019. As used herein, Process A refers to a conventional method of encapsulating mRNA by mixing mRNA with a mixture of lipids, without first pre-forming the lipids into lipid nanoparticles, as described in US 2016/0038432. As used herein, Process B refers to a process of encapsulating messenger RNA (mRNA) by mixing pre-formed lipid nanoparticles with mRNA, as described in US 2018/0153822.

Briefly, the process of preparing mRNA-loaded lipid liposomes includes a step of heating one or more of the solutions (i.e., applying heat from a heat source to the solution) to a temperature (or to maintain at a temperature) greater than ambient temperature, the one more solutions being the solution comprising the pre-formed lipid nanoparticles, the solution comprising the mRNA and the mixed solution comprising the lipid nanoparticle encapsulated mRNA. In some embodiments, the process includes the step of heating one or both of the mRNA solution and the pre-formed lipid nanoparticle solution, prior to the mixing step. In some embodiments, the process includes heating one or more one or more of the solution comprising the pre-formed lipid nanoparticles, the solution comprising the mRNA and the solution comprising the lipid nanoparticle encapsulated mRNA, during the mixing step. In some embodiments, the process includes the step of heating the lipid nanoparticle encapsulated mRNA, after the mixing step. In some embodiments, the temperature to which one or more of the solutions is heated (or at which one or more of the solutions is maintained) is or is greater than about 30 °C, 37 °C, 40 °C, 45 °C, 50 °C, 55 °C, 60 °C, 65 °C, or 70 °C. In some embodiments, the temperature to which one or more of the solutions is heated ranges from about 25-70 °C, about 30-70 °C, about 35-70 °C, about 40-70 °C, about 45-70 °C, about 50-70 °C, or about 60-70 °C. In some embodiments, the temperature greater than ambient temperature to which one or more of the solutions is heated is about 65 °C.

Various methods may be used to prepare an mRNA solution suitable for the present invention. In some embodiments, mRNA may be directly dissolved in a buffer solution described herein. In some embodiments, an mRNA solution may be generated by mixing an mRNA stock solution with a buffer solution prior to mixing with a lipid solution for encapsulation. In some embodiments, an mRNA solution may be generated by mixing an mRNA stock solution with a buffer solution immediately before mixing with a lipid solution for encapsulation. In some embodiments, a suitable mRNA stock solution may contain mRNA in water at a concentration at or greater than about 0.2 mg/ml, 0.4 mg/ml, 0.5 mg/ml, 0.6 mg/ml, 0.8 mg/ml, 1.0 mg/ml, 1.2 mg/ml, 1.4 mg/ml, 1.5 mg/ml, or 1.6 mg/ml, 2.0 mg/ml, 2.5 mg/ml, 3.0 mg/ml, 3.5 mg/ml, 4.0 mg/ml, 4.5 mg/ml, or 5.0 mg/ml.

In some embodiments, an mRNA stock solution is mixed with a buffer solution using a pump. Exemplary pumps include but are not limited to gear pumps, peristaltic pumps and centrifugal pumps.

Typically, the buffer solution is mixed at a rate greater than that of the mRNA stock solution. For example, the buffer solution may be mixed at a rate at least 1x, 2x, 3x, 4x, 5x, 6x, 7x, 8x, 9x, 10x, 15x, or 20x greater than the rate of the mRNA stock solution. In some embodiments, a buffer solution is mixed at a flow rate ranging between about 100-6000 ml/minute (e.g., about 100-300 ml/minute, 300-600 ml/minute, 600-1200 ml/minute, 1200-2400 ml/minute, 2400-3600 ml/minute, 3600-4800 ml/minute, 4800-6000 ml/minute, or 60-420 ml/minute). In some embodiments, a buffer solution is mixed at a flow rate of or greater than about 60 ml/minute, 100 ml/minute, 140 ml/minute, 180 ml/minute, 220 ml/minute, 260 ml/minute, 300 ml/minute, 340 ml/minute, 380 ml/minute, 420 ml/minute, 480 ml/minute, 540 ml/minute, 600 ml/minute, 1200 ml/minute, 2400 ml/minute, 3600 ml/minute, 4800 ml/minute, or 6000 ml/minute.

In some embodiments, an mRNA stock solution is mixed at a flow rate ranging between about 10-600 ml/minute (e.g., about 5-50 ml/minute, about 10-30 ml/minute, about 30-60 ml/minute, about 60-120 ml/minute, about 120-240 ml/minute, about 240-360 ml/minute, about 360-480 ml/minute, or about 480-600 ml/minute). In some embodiments, an mRNA stock solution is mixed at a flow rate of or greater than about 5 ml/minute, 10 ml/minute, 15 ml/minute, 20 ml/minute, 25 ml/minute, 30 ml/minute, 35 ml/minute, 40 ml/minute, 45 ml/minute, 50 ml/minute, 60 ml/minute, 80 ml/minute, 100 ml/minute, 200 ml/minute, 300 ml/minute, 400 ml/minute, 500 ml/minute, or 600 ml/minute.

According to the present invention, a lipid solution contains a mixture of lipids suitable to form lipid nanoparticles for encapsulation of mRNA. In some embodiments, a suitable lipid solution is ethanol based. For example, a suitable lipid solution may contain a mixture of desired lipids dissolved in pure ethanol (i.e., 100% ethanol). In another embodiment, a suitable lipid solution is isopropyl alcohol based. In another embodiment, a suitable lipid solution is dimethylsulfoxide-based. In another embodiment, a suitable lipid solution is a mixture of suitable solvents including, but not limited to, ethanol, isopropyl alcohol and dimethylsulfoxide.

A suitable lipid solution may contain a mixture of desired lipids at various concentrations. For example, a suitable lipid solution may contain a mixture of desired lipids at a total concentration of or greater than about 0.1 mg/ml, 0.5 mg/ml, 1.0 mg/ml, 2.0 mg/ml, 3.0 mg/ml, 4.0 mg/ml, 5.0 mg/ml, 6.0 mg/ml, 7.0 mg/ml, 8.0 mg/ml, 9.0 mg/ml, 10 mg/ml, 15 mg/ml, 20 mg/ml, 30 mg/ml, 40 mg/ml, 50 mg/ml, or 100 mg/ml. In some embodiments, a suitable lipid solution may contain a mixture of desired lipids at a total concentration ranging from about 0.1-100 mg/ml, 0.5-90 mg/ml, 1.0-80 mg/ml, 1.0-70 mg/ml, 1.0-60 mg/ml, 1.0-50 mg/ml, 1.0-40 mg/ml, 1.0-30 mg/ml, 1.0-20 mg/ml, 1.0-15 mg/ml, 1.0-10 mg/ml, 1.0-9 mg/ml, 1.0-8 mg/ml, 1.0-7 mg/ml, 1.0-6 mg/ml, or 1.0-5 mg/ml. In some embodiments, a suitable lipid solution may contain a mixture of desired lipids at a total concentration up to about 100 mg/ml, 90 mg/ml, 80 mg/ml, 70 mg/ml, 60 mg/ml, 50 mg/ml, 40 mg/ml, 30 mg/ml, 20 mg/ml, or 10 mg/ml.

Any desired lipids may be mixed at any ratios suitable for encapsulating mRNAs. In some embodiments, a suitable lipid solution contains a mixture of desired lipids including cationic lipids, helper lipids (e.g. non cationic lipids and/or cholesterol lipids), amphiphilic block copolymers (e.g. poloxamers) and/or PEGylated lipids. In some embodiments, a suitable lipid solution contains a mixture of desired lipids including one or more cationic lipids, one or more helper lipids (e.g. non cationic lipids and/or cholesterol lipids) and one or more PEGylated lipids

In certain embodiments, provided compositions comprise a liposome wherein the mRNA is encapsulated within the same liposome. For example, during preparation of the compositions of the present invention, cationic liposomes may associate with the mRNA through electrostatic interactions.

As defined in the claims, the compositions and methods of the invention comprise mRNA encapsulated in a liposome. In some embodiments, the one or more mRNA species may be encapsulated in the same liposome. In some embodiments, the one or more mRNA species may be encapsulated in different liposomes. In some embodiments, the mRNA is encapsulated in one or more liposomes, which differ in their lipid composition, molar ratio of lipid components, size, charge (Zeta potential), targeting ligands and/or combinations thereof. In some embodiments, the one or more liposome may have a different composition of cationic lipids, neutral lipid, PEG-modified lipid and/or combinations thereof. In some embodiments the one or more lipsomes may have a different molar ratio of cationic lipid, neutral lipid, cholesterol and PEG-modified lipid used to create the liposome.

The process of incorporation of a desired mRNA into a liposome is often referred to as "loading". Exemplary methods are described in Lasic, et al., FEBS Lett., 312: 255-258, 1992. The liposome-incorporated nucleic acids (interchangeably used with the term mRNA-loaded lipid nanoparticles) may be completely or partially located in the interior space of the liposome, within the bilayer membrane of the liposome, or associated with the exterior surface of the liposome membrane. The incorporation of a nucleic acid into liposomes is also referred to herein as "encapsulation" wherein the nucleic acid is entirely contained within the interior space of the liposome. The purpose of incorporating an mRNA into a transfer vehicle, such as a liposome, is often to protect the nucleic acid from an environment which may contain enzymes or chemicals that degrade nucleic acids and/or systems or receptors that cause the rapid excretion of the nucleic acids. Accordingly, in some embodiments, a suitable delivery vehicle is capable of enhancing the stability of the mRNA contained therein and/or facilitate the delivery of mRNA to the target cell or tissue.

In some embodiments, mRNA is mixed with preformed lipid nanoparticles or liposomes to form mRNA-loaded LNPs.

### Nanoparticle Size

Suitable liposomes or other nanoparticles in accordance with the present invention may be made in various sizes. In some embodiments, a suitable nanoparticle has a size of or less than about 100 nm (e.g., of or less than about 90 nm, 80 nm, 70 nm, 60 nm, 50 nm, 40 nm, 30 nm, or 20 nm). In some embodiments, the nanoparticle has a size of or less than about 60 nm (e.g., of or less than about 55 nm, of or less than about 50 nm, of or less than about 45 nm, of or less than about 40 nm, of or less than about 35 nm, of or less than about 30 nm, or of or less than about 25 nm). In some embodiments, a suitable nanoparticle has a size ranging from about 10 - 100 nm (e.g., ranging from about 10 - 90 nm, 10 - 80 nm, 10 - 70 nm, 10 - 60 nm, 10 - 50 nm, 10-40 nm, or 10-30 nm).

A variety of alternative methods known in the art are available for sizing of a population of liposomes. One such sizing method is described in U.S. Pat. No. 4,737,323. Sonicating a liposome suspension either by bath or probe sonication produces a progressive size reduction down to small ULV less than about 0.05 microns in diameter. Homogenization is another method that relies on shearing energy to fragment large liposomes into smaller ones. In a typical homogenization procedure, MLV are recirculated through a standard emulsion homogenizer until selected liposome sizes, typically between about 0.1 and 0.5 microns, are observed. The size of the liposomes may be determined by quasi-electric light scattering (QELS) as described in Bloomfield, Ann. Rev. Biophys. Bioeng., 10:421-150 (1981). Average liposome diameter may be reduced by sonication of formed liposomes. Intermittent sonication cycles may be alternated with QELS assessment to guide efficient liposome synthesis.

### Pharmaceutical Compositions and Administration

To facilitate expression of mRNA *in vivo,* delivery vehicles such as liposomes can be formulated in combination with one or more additional nucleic acids, carriers, targeting ligands or stabilizing reagents, or in pharmacological compositions where it is mixed with suitable excipients. Techniques for formulation and administration of drugs may be found in "Remington's Pharmaceutical Sciences," Mack Publishing Co., Easton, Pa., latest edition.

Provided mRNA (naked or nanoparticle-encapsulated or associated), and compositions containing the same, may be administered and dosed in accordance with current medical practice, taking into account the clinical condition of the subject, the site and method of administration, the scheduling of administration, the subject's age, sex, body weight and other factors relevant to clinicians of ordinary skill in the art. mRNA (naked or nanoparticle-encapsulated or associated), and compositions containing the same, may be administered into the eye of a subject via intravitreal, intracameral, subconjunctival, subtenon, retrobulbar, topical, suprachoroidal and/or posterior juxtascleral administration. In some embodiments the mRNA compositions are delivered via injection into the eye. In some embodiments the mRNA compositions are delivered via intravitreal injection into the eye. In some embodiments the mRNA compositions are delivered via subconjunctival injection into the eye. In some embodiments the mRNA compositions are delivered via suprachoroidal injection into the eye. In some embodiments, the mRNA compositions are delivered via surgical incision. In some embodiments, the mRNA compositions are delivered via micro-cannulation. In some embodiments, the mRNA compositions are delivered via intracameral administration. In some embodiments, the mRNA compositions are delivered via subconjunctival administration. In some embodiments, the mRNA compositions are delivered via subtenon administration. In some embodiments, the mRNA compositions are delivered via retrobulbaradministration. In some embodiments the mRNA compositions are delivered via topical administration.

In some embodiments, the mRNA compositions comprise mRNA-loaded lipid nanoparticles as described in the preceding sections. In some embodiments, the mRNA-loaded lipid nanoparticles are formulated in liquid suspensions for delivery into the eye. In some embodiments, the mRNA compositions are delivered in a suspension volume of 0.5 µl - 100 µl per eye. In some embodiments, the mRNA compositions are delivered in a suspension volume of 1 µl - 100 µl per eye. In some embodiments, the mRNA compositions are delivered in a suspension volume of 2 µl - 100 µl per eye, 5 µl - 100 µl per eye, 10 µl - 100 µl per eye, 20 µl - 100 µl per eye, or 1 µl - 50 µl per eye. In some embodiments the mRNA composition is delivered in a solution having a total volume of 1 µl, 2 µl , 3 µl, 4 µl , 5 µl , 6 µl, 7 µl , 8 µl , 9 µl, 10 µl, 15 µl , 20 µl , 25 µl , 30 µl , 40 µl, 50 µl or 100 µl per eye. Typical volumes administered to human subjects by intravitreal injection range from about 30 µl to about 100 µl. For example, volumes of about 30 µl, about 50 µl, about 70 µl and about 100 µl are commonly administered to human subjects by intravitreal injection. Volumes of about 30 µl are suitable for intravitreal administration to infants.

In some embodiments, the mRNA compositions delivered to the eye have a concentration of about 0.001 mg/ml, 0.01 mg/ml, 0.02 mg/ml, 0.03 mg/ml, 0.04 mg/ml, 0.05 mg/ml, 0.06 mg/ml, 0.07 mg/ml, 0.08 mg/ml, 0.09 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1 mg/ml, 2 mg/ml, 3 mg/ml, 4 mg/ml, 5 mg/ml, 6 mg/ml, 7 mg/ml, 8 mg/ml, 9 mg/ml, about 10 mg/ml or about 50 mg/ml, or about 100 mg/ml. In some embodiments the mRNA compositions delivered to the eye have a concentration of about 0.001 mg/ml. In some embodiments the mRNA compositions delivered to the eye have a concentration of about 0.005 mg/ml. In some embodiments the mRNA compositions delivered to the eye have a concentration of about 0.006 mg/ml. In some embodiments the mRNA compositions delivered to the eye have a concentration of about 0.007 mg/ml. In some embodiments the mRNA compositions delivered to the eye have a concentration of about 0.008 mg/ml. In some embodiments the mRNA compositions delivered to the eye have a concentration of about 0.009 mg/ml. In some embodiments the mRNA compositions delivered to the eye have a concentration of about 0.01 mg/ml. In some embodiments the mRNA compositions delivered to the eye have a concentration of about 0.02 mg/ml. In some embodiments the mRNA compositions delivered to the eye have a concentration of about 0.05 mg/ml. In some embodiments, the mRNA compositions delivered to the eye have a concentration of about 0.1 mg/ml. In some embodiments, the mRNA compositions delivered to the eye have a concentration of about 0.5 mg/ml. In some embodiments, the mRNA compositions delivered to the eye have a concentration of about 1 mg/ml. In some embodiments, the mRNA compositions delivered to the eye have a concentration of about 10 mg/ml. In some embodiments, the mRNA compositions delivered to the eye have a concentration of about 50 mg/ml. In some embodiments the mRNA compositions delivered to the eye have a concentration of about 100 mg/ml.

mRNA concentrations ranging from about 0.5 mg/ml to about 0.8 mg/ml are particularly suitable for injection. An exemplary injectable mRNA composition for use with the invention has an mRNA concentration of about 0.6 mg/ml.

The "effective amount" or "an effective therapeutic dose" for the purposes herein may be determined by such relevant considerations as are known to those of ordinary skill in experimental clinical research, pharmacological, clinical and medical arts. In some embodiments, an effective dose of mRNA delivered to an eye is 5 µg, 10 µg, 20 µg, 50 µg or about 100 µg of mRNA.

The inventors demonstrate herein that it is possible to successfully extrapolate from an mRNA dose that results in expression of the mRNA-encoded protein throughout the retina in a small rodent model such as a mouse to determine an effective amount of mRNA to achieve corresponding protein expression in the eye of a much larger mammal such as a rabbit. Extrapolation to the even larger eyes of humans is equally possible.

As defined in the claims, an effective dose of mRNA delivered to the human eye ranges from about from about 5 µg to about 100 µg. For example, an effective dose of mRNA delivered to the human eye may range from about 10 µg to about 80 µg. A dose ranging from about 30 µg to about 60 µg may be effective for a wide range of therapeutic applications. An effective dose of mRNA suitable for treating an infant may be 50% of the adult dose.

Higher doses may be required for the treatment of diseases or disorders affecting the posterior retinal layers (i.e., the layers furthest from the vitreous humor) and other tissues of the posterior eye, such as the outer nuclear layer (ONL), the inner segment photoreceptors (IS), the outer segment photoreceptors (OS), the retinal pigmented epithelium layer (RPE) of the retinal tissue, the choroid, and/or the sclera. For instance, a dose from about 40 µg to about 100 µg may be suitable for treating diseases or disorders affecting the posterior retinal layers and/or other tissues of the posterior eye. A dose from about 50 µg to about 80 µg may be particularly effective for treating these diseases or disorders. Examples of diseases and disorders affecting posterior retinal layers or other tissues of the posterior eye include age-related macular degeneration (AMD), cytomegalovirus (CMV) retinitis, Leber's congenital amaurosis, Stargardt disease, Usher disease, chorioretinitis, retinal detachment, uveitis, uvetic macular edema, cyclitis, choroiditis, diffuse uveitis and scleritis.

Provided methods of the present invention contemplate single as well as multiple administrations of a therapeutically effective amount of mRNA or a composition described herein. mRNA or a composition described herein can be administered at regular intervals, depending on the nature, severity and extent of the subject's condition. In some embodiments, a therapeutically effective amount of mRNA or a composition described herein may be administered periodically at regular intervals (e.g., once every year, once every six months, once every five months, once every four months, once every three months, bimonthly (once every two months), monthly (once every month), once every three weeks, biweekly (once every two weeks), weekly, once every three days, once every two days, daily or continuously). In some embodiments, mRNA or a composition described herein may be administered at variable intervals. In some embodiments, a suitable amount and dosing regimen is one that results in protein (e.g., antibody) expression or activity in the eye. As defined in the claims, the expression and/or activity of the protein is detected in the posterior region of the eye. In some embodiments, the expression and/or activity of the protein is detected in both the posterior and anterior regions of the eye. In some embodiments, the expression and/or activity of the protein is detected by blood sampling. In some embodiments, the expression and/or activity of the protein is detected by sampling a vitreous humor.

The methods and compositions provided herein result in delivery of the mRNA into the posterior segment of the eye, namely the retina, the choroid or the sclera. In some embodiments, the expression and/or activity of the protein is detected in corneal cells, scleral cells, choroid plexus epithelial cells, ciliary body cells, retinal cells, and/or vitreous humor. In some embodiments, the delivery of the mRNA using the methods and compositions of the invention result in expression of the mRNA-encoded protein inside the retina. Delivery of lipid encapsulated mRNA as described herein results in expression of the mRNA-encoded protein in one or more cells located in the outer nuclear layer (ONL), the inner segment photoreceptors (IS), the outer segment photoreceptors (OS), the retinal pigmented epithelium layer (RPE) of the retinal tissue, the choroid, and/or the sclera of the eye. In some embodiments, the methods and compositions for delivery of the mRNA in the eye as described herein, result in expression of the mRNA-encoded protein in the inner segment photoreceptors (IS) of the retina. In some embodiments, the methods and compositions for delivery of the mRNA in the eye as described herein, result in expression of the mRNA-encoded protein in the outer segment photoreceptors (OS) of the retina. In some embodiments, the methods and compositions for delivery of the mRNA in the eye as described herein, result in expression of the mRNA-encoded protein in the retinal pigmented epithelium layer (RPE) of the retina. In some embodiments, the methods and compositions for delivery of the mRNA in the eye as described herein, result in expression of the mRNA-encoded protein in the choroid. In some embodiments, the methods and compositions for delivery of the mRNA in the eye as described herein, result in expression of the mRNA-encoded protein in the sclera.

In some embodiments, the expression and/or activity of the protein is detectable about 6 hours, 12 hours, 18 hours, 24 hours, 2 days, 3 days, 4 days, 5 days, 6 days, 1 week, 2 weeks, 3 weeks, 4 weeks, 1 month, 2 months, 3 months, 4 months, 5 months, 6 months or longer after a single administration. In some embodiments, the amount administered is effective to achieve at least some stabilization, improvement or elimination of symptoms and other indicators as are selected as appropriate measures of disease progress, regression or improvement by those of skill in the art.

Treatment of various diseases or disorders are contemplated with the methods and compositions described herein. As defined in the claims, expression of the protein encoded by the mRNA that is administered to a subject results in treating a disease or disorder or a condition relating to the eye of the subject. Exemplary diseases or disorders comprise uveitis, cyclitis, diffuse uveitis, choroiditis, Stargardt disease, age related macular degeneration. Various diseases affect different layers of the retina. The surprisingly robust protein expression in the disease relevant retinal cells using the methods and composition of the invention leads to new avenues of treatment of such diseases. The methods and compositions can be employed to express mRNA encoding a variety of proteins. In some embodiments, the mRNA encodes a protein or a peptide selected from a group consisting of an ocular protein or a peptide, a vaccine, an antibody or a fragment thereof, a hormone, a structural protein or peptide, an extracellular matrix protein or peptide, a vascular protein or peptide, an anti-tumor protein or peptide, an angiogenic protein or peptide, an anti-angiogenic protein or peptide, an antioxidant protein or peptide, a receptor protein or peptide, a signaling protein or peptide, a transcription factor and an enzyme. In some embodiments, the mRNA encodes an ocular protein or a peptide selected from a group consisting of ADAM metallopeptidase domain 9, adhesins, ATP synthase, bestrophin 1, cadherins, chemokines, ciliary neurotrophic factor, collagens, complement factors, cytochromes, IGF, metalloproteinases, mitofusin, NADH dehydrogenase, OPA1, PDGF, peripherin 2, retinoschisin, SOD2, thrombospondin receptor, and vascular endothelial growth factor (VEGF, including but not limited to VEGF-A, VEGF-B, VEGF-C and other isoforms). In some embodiments, the mRNA encodes an antibody or a fragment thereof, that binds to ADAM metallopeptidase domain 9, adhesins, ATP synthase, bestrophin 1, cadherins, chemokines, ciliary neurotrophic factor, collagens, complement factors, cytochromes, IGF, metalloproteinases, mitofusin, NADH dehydrogenase, OPA1, PDGF, peripherin 2, retinoschisin, SOD2, thrombospondin receptor, or vascular endothelial growth factor (VEGF, including VEGF-A, VEGF-B, VEGF-C and other isoforms). In some embodiments, the mRNA encodes an antibody or a fragment thereof that binds to VEGF (including VEGF-A, VEGF-B, VEGF-C and other isoforms). In some embodiments, administering the composition results in a decrease or amelioration of one or more symptoms associated with the ocular disease or disorder.

### EXAMPLES

### Lipid Materials

The formulations described in the following Examples, unless otherwise specified, contain a multi-component lipid mixture of varying ratios employing one or more cationic lipids, helper lipids (e.g. non-cationic lipids and/or cholesterol lipids), and PEGylated lipids designed to encapsulate various nucleic acid-based materials. Cationic lipids can include (but not exclusively) DOTAP (1,2-dioleyl-3-trimethylammonium propane), DODAP (1,2-dioleyl-3-dimethylammonium propane) , DOTMA (1,2-di-O-octadecenyl-3-trimethylammonium propane), DLinDMA (Heyes, J.; Palmer, L.; Bremner, K.; MacLachlan, I. "Cationic lipid saturation influences intracellular delivery of encapsulated nucleic acids" J. Contr. Rel. 2005, 107, 276-287), DLin-KC2-DMA (Semple, S.C. et al. "Rational Design of Cationic Lipids for siRNA Delivery" Nature Biotech. 2010, 28, 172-176), C12-200 (Love, K.T. et al. "Lipid-like materials for low-dose in vivo gene silencing" PNAS 2010, 107, 1864-1869), MD1 (3,6-bis(4-(bis(2-hydroxydodecyl)amino)butyl)piperazine-2,5-dione), cKK-E12, HGT5000, HGT5001, HGT4003, ICE, dialkylamino-based, imidazole-based, guanidinium-based, etc. Helper lipids can include (but not exclusively) DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), DOPG (,2-dioleoyl-sn-glycero-3-phospho-(1'-rac-glycerol)), cholesterol, etc. The PEGylated lipids can include (but not exclusively) a poly(ethylene) glycol chain of up to 5 kDa in length covalently attached to a lipid with alkyl chain(s) of C6-C20 length. Polyethyleneimine can be linear or branched. For branched PEI, 25 kDa is preferred but not exclusive.

### mRNA Materials

For illustration purposes, the mRNA used in the following examples encode either OTC (ornithine carbamoyltransferase) or EGFP (enhanced green fluorescent protein). The mRNA sequences used in the following examples correspond to the following cDNA sequences.
OTC cDNA: 
GGACAGATCGCCTGGAGACGCCATCCACGCTGTTTTGACCTCCATAGAAGACACCG
GGACCGATCCAGCCTCCGCGGCCGGGAACGGTGCATTGGAACGCGGATTCCCCGTG
CCAAGAGTGACTCACCGTCCTTGACACGATGCTGTTCAACCTTCGGATCTTGCTGAA
CAACGCTGCGTTCCGGAATGGTCACAACTTCATGGTCCGGAACTTCAGATGCGGCCA
GCCGCTCCAGAACAAGGTGCAGCTCAAGGGGAGGACCTCCTCACCCTGAAAAACT
TCACCGGAGAAGAGATCAAGTACATGCTGTGGCTGTCAGCCGACCTCAAATTCCGG
ATCAAGCAGAAGGGCGAATACCTTCCTTTGCTGCAGGGAAAGTCCCTGGGGATGAT
CTTCGAGAAGCGCAGCACTCGCACTAGACTGTCAACTGAAACCGGCTTCGCGCTGCT
GGGAGGACACCCCTGCTTCCTGACCACCCAAGATATCCATCTGGGTGTGAACGAATC
CCTCACCGACACAGCGCGGGTGCTGTCGTCCATGGCAGACGCGGTCCTCGCCCGCGT
GTACAAGCAGTCTGATCTGGACACTCTGGCCAAGGAAGCCTCCATTCCTATCATTAA
TGGATTGTCCGACCTCTACCATCCCATCCAGATTCTGGCCGATTATCTGACTCTGCAA
GAACATTACAGCTCCCTGAAGGGGCTTACCCTTTCGTGGATCGGCGACGGCAACAA
CATTCTGCACAGCATTATGATGAGCGCTGCCAAGTTTGGAATGCACCTCCAAGCAGC
GACCCCGAAGGGATACGAGCCAGACGCCTCCGTGACGAAGCTGGCTGAGCAGTACG
CCAAGGAGAACGGCACTAAGCTGCTGCTCACCAACGACCCTCTCGAAGCCGCCCAC
GGTGGCAACGTGCTGATCACCGATACCTGGATCTCCATGGGACAGGAGGAGGAAAA
GAAGAAGCGCCTGCAAGCATTTCAGGGGTACCAGGTGACTATGAAAACCGCCAAGG
TCGCCGCCTCGGACTGGACCTTCTTGCACTGTCTGCCCAGAAAGCCCGAAGAGGTGG
ACGACGAGGTGTTCTACAGCCCGCGGTCGCTGGTCTTTCCGGAGGCCGAAAACAGG
AAGTGGACTATCATGGCCGTGATGGTGTCCCTGCTGACCGATTACTCCCCGCAGCTG
CAGAAACCAAAGTTCTGACGGGTGGCATCCCTGTGACCCCTCCCCAGTGCCTCTCCT
GGCCCTGGAAGTTGCCACTCCAGTGCCCACCAGCCTTGTCCTAATAAAATTAAGTTG
CATCAAGCT (SEQ ID NO:1)
EGFP cDNA:

GGACAGATCGCCTGGAGACGCCATCCACGCTGTTTTGACCTCCATAGAAGACACCG
GGACCGATCCAGCCTCCGCGGCCGGGAACGGTGCATTGGAACGCGGATTCCCCGTG
CCAAGAGTGACTCACCGTCCTTGACACGATGGGAAAGCCTATCCAAACCCCCTCCT
CGGCCTTGACTCCACTCGCGATCCCCCAGTGGCGACCATTGTCTCCAAGGGCGAAGA

ATTATTCACCGGAGTCGTGCCTATCCTCGTGGAACTGGATGGCGACGTGAACGGACA
CAAATTCAGCGTGTCGGGAGAGGGGGAAGGGGACGCCACTTATGGAAAGCTCACCC
TGAAGTTCATTTGCACTACTGGAAAGCTCCCCGTGCCTTGGCCCACCCTTGTGACCA
CCCTGACCTACGGCGTGCAGTGCTTTTCCCGGTACCCGGACCACATGAAGCAACACG
ACTTCTTCAAGAGCGCTATGCCGGAAGGCTACGTGCAGGAGCGGACGATATTCTTCA
AGGATGACGGGAATTACAAAACCCGCGCCGAAGTCAAGTTTGAGGGCGATACCCTT
GTGAACAGAATCGAGCTGAAGGGTATTGACTTCAAGGAGGACGGAAACATCCTGGG
CCACAAGCTCGAGTACAACTACAACTCCCATAACGTCTACATTATGGCAGACAAGC
AGAAGAACGGTATCAAGGTCAACTTCAAGATTAGGCATAACATCGAGGACGGCTCG
GTGCAGCTCGCCGACCATTACCAGCAAAATACCCCGATTGGGGACGGACCGGTGCT
GCTGCCGGACAACCACTACTTGAGCACTCAAAGCGCGCTGTCAAAGGATCCGAACG
AAAAGCGCGATCACATGGTCCTGCTGGAGTTCGTGACTGCCGCCGGAATCACACTG
GGAATGGACGAATTGTACAAATAACGGGTGGCATCCCTGTGACCCCTCCCCAGTGC
CTCTCCTGGCCCTGGAAGTTGCCACTCCAGTGCCCACCAGCCTTGTCCTAATAAAAT
TAAGTTGCATCAAGCT (SEQ ID NO:2)

The mRNA sequences shown above were used as proof of concept and are not intended to be limiting with respect to the mRNA that can be delivered using the method described herein.

### Example 1. Analysis of protein expression in mice following a single intravitreal injection of mRNA-loaded lipid nanoparticles

This example illustrates exemplary methods of administering mRNA-loaded lipid nanoparticles. Also shown are methods for analyzing delivered mRNA and subsequently expressed protein in target tissues (*e.g.* the retina) *in vivo.* mRNA encoding OTC or EGFP were formulated in lipid nanoparticles (LNPs) comprising the cationic lipid cKK-E12, the non-cationic lipid DOPE, cholesterol and the PEG-modified lipid DMG-PEG2K at a molar ratio of 40:30:25:5. LNPs had a lipid: mRNA ratio (designated as N/P ratio) of 4. The mixing was done under steady pressure using a pump system. The mRNA-loaded LNPs (mRNA-LNPs) were less than 100 µm in diameter. Unless otherwise stated, the following examples utilize the same formulation described in this paragraph.

### ELISA Assay for Detecting Protein Expression in Mouse Retina

Male CD-1 mice of approximately 6-8 weeks of age were injected intravitreally with lipid nanoparticles comprising mRNA encoding either OTC (ornithine carbamoyl transferase) or EGFP (enhanced green fluorescent protein). For each of OTC- or EGF mRNA-loaded lipid nanoparticle (OTC-LNP and EGFP-LNP respectively) administration studies, mice were divided into 6 groups of 5 mice per group, as indicated in Table 1. Group 1 and 7 mice received Phosphate Buffered Saline (PBS) as control. 5 additional groups of mice received 1 µg, 0.5 µg, 0.25 µg, 0.125 µg and 0.0625 µg of OTC mRNA-LNP. Similarly, 5 additional groups of mice received 1 µg, 0.5 µg, 0.25 µg, 0.125 µg and 0.0625 µg of EGFP mRNA-LNP as shown in Table 1. Animals were anesthetized with either ketamine/xylazine injection or isoflurane inhalation. The eyes were locally anesthetized with tropical proparacaine and cleaned with Betadine solution. mRNA-loaded lipid nanoparticles were injected into each eye via intravitreal injection (1 µl volume per eye). Table 1 provides detailed layout of the study design. All of the administered doses were well tolerated by the test animals.

**Table 1.**

| Group No. | No. of Animals | mRNA | Dose (µg/eye) | Conc. (mg/mL) | Dose Volume | Terminal Time Point |
|---|---|---|---|---|---|---|
| 1 | 5 | None (PBS) | 0.0 | 0.0 | 1.0 µL/eye | 24 hr post-administration |
| 2 | 5 | OTC | 1.0 | 1.0 | | |
| 3 | 5 | OTC | 5.0 | 0.5 | | |
| 4 | 5 | OTC | 0.25 | 0.25 | | |
| 5 | 5 | OTC | 0.125 | 0.125 | | |
| 6 | 5 | OTC | 0.0625 | 0.0625 | | |
| 7 | 5 | PBS | 0.0 | 0.0 | | |
| 8 | 5 | EGFP | 1.0 | 1.0 | | |
| 9 | 5 | EGFP | 5.0 | 0.5 | | |
| 10 | 5 | EGFP | 0.25 | 0.25 | | |
| 11 | 5 | EGFP | 0.125 | 0.125 | | |
| 12 | 5 | EGFP | 0.0625 | 0.0625 | | |

Twenty four hours after mRNA-loaded lipid nanoparticles administration, all animals were euthanized by CO₂ asphyxiation followed by thoracotomy. Both eyes were harvested and submerged in PBS. Retina was isolated from each eye and snap frozen in liquid nitrogen. Protein was extracted from frozen retinal samples from of each animal and subjected to ELISA assay for OTC and EGFP protein expression. Standard ELISA and immunofluorescence procedures followed using commercially available assay kits and systems.

Fig. 1 illustrates ELISA detection of expressed OTC protein in mouse retina. At 24 hours post administration, a clear dose-dependent protein expression was noted in the samples, as shown in Fig. 1. Surprisingly, OTC expression was detectable at even the lowest mRNA dose of 0.0625 µg. This indicates that the delivery of mRNA was effective at a low dose.

Figs. 2A-B illustrate ELISA detection of expressed EGFP protein in mouse retina. Similar to the results shown in Fig. 1, EGFP expression also showed a dose dependent expression of EGFP in mouse retina. The expression levels (Y-axis) are presented in a linear scale (Fig. 2A) as well as logarithmic scale (Fig. 2B), both of which illustrate that even at the lowest dose of mRNA (0.0625 µg) detectable expression was present at 24 hours after the administration. These results indicate successful and effective delivery of low dose mRNA to the retina by intravitreal injection of lipid-encapsulated leading to mRNA-encoded protein expression in the retina by intravitreal administration of a low dose mRNA. In cases of both OTC and EGFP expression, as illustrated in Fig. 1 and Figs 2A-B respectively, administration of 0.0625 µg, 0.125 µg, 0.25 µg, 0.5 µg and 1 µg mRNA led to robust and dose dependent protein expression in the retina.

### Immunohistochemistry for Detecting Protein Expression in Mouse Retina

In this study, CD1 mice were administered either PBS or OTC -LNP or EGFP-LNP as indicated in Table 2. Administration of PBS or OTC -LNP or EGFP-LNP was performed by intravitreal injection in a 5 µl total volume per eye (Table 2).

**Table 2.**

| Group No. | No. of Animals | mRNA | Dose (µg/eye) | Conc. (mg/mL) | Dose Volume | Terminal Time Point |
|---|---|---|---|---|---|---|
| 1 | 3 | None (PBS) | 0.0 | 0.0 | | |
| 2 | 3 | OTC | 5.0 | 1.0 | | |
| 3 | 3 | EGFP | 5.0 | 1.0 | 5.0 µL/eye | 24 hr post-administration |
| 4 | 2 | None (PBS) | 0.0 | 0.0 | | |
| 5 | 2 | OTC | 5.0 | 1.0 | | |
| 6 | 2 | EGFP | 5.0 | 1.0 | | |

At 24 hours after the administration, mice were euthanized. For groups 1-3 (as described in Table 2), the left eyes (intact) of each animal was placed Davidson's Fixative. [Davidson's fixative (900 mL solution): 100 mL glacial acetic acid, 300 mL of 95% ethanol, 200 mL of 10% neutral buffered formalin, and 300 mL distilled water]. The right eye (intact) of each animal was placed 10% neutral buffered formalin (NBF). Immunofluorescence was performed by standard procedures using fluorescence tagged antibodies directed to anti-OTC and anti-EGFP antibodies for detection of OTC and EGFP proteins respectively.

Fig. 3 shows a representative set of immunofluorescence study indicating that the above described method resulted in robust retinal delivery. Following a single 1.0 µg dose of mRNA encoding OTC or mRNA encoding EGFP, specific immunofluorescence of OTC protein (upper panel) and EGFP protein (lower panel) was detected in retinal tissue at 24 hours post administration. This result shows expansive delivery capabilities of an mRNA-loaded lipid nanoparticle as protein production was detected throughout the retina, capable of reaching deep into the posterior of the eye (Fig. 3).

### Example 2. Delivery of mRNA-Loaded Lipid Nanoparticles to Retinal Tissue Layers

This example illustrates that administration of mRNA-loaded lipid nanoparticles by the methods of the invention resulted in protein expression in multiple retinal tissue layers.

mRNA encoding OTC were formulated in lipid nanoparticles (LNPs) comprising the cationic lipid cKK-E12, the non-cationic lipid DOPE, cholesterol and the PEG-modified lipid DMG-PEG2K, as described above. New Zealand white rabbits weighing approximately 1.5 to 1.7 kg were injected with lipid nanoparticles comprising mRNA encoding OTC (OTC-LNP). While animals are anesthetized with 30-40 mg/kg ketamine/~0.5-10 mg/kg xylazine injection, they received an injection containing the mRNA-loaded lipid nanoparticles into each eye via a single intravitreal injection. The eyes were locally anesthetized with tropical proparacaine and cleaned with Betadine solution. Animals were dosed and treated according to Table 3. All of the administered doses were well tolerated by the test animals.

**Table 3.**

| Group No. | No. of Animals | mRNA | Dose (µg/eye) | Conc. (mg/mL) | Dose Volume | Terminal Time Point |
|---|---|---|---|---|---|---|
| 1 | 5 | None (PBS) | 0.0 | 0.0 | | 24 hr post-administration |
| 2 | 5 | OTC | 50 | 1.0 | | |
| 3 | 5 | OTC | 25 | 0.5 | 50 µL/eye | |
| 4 | 5 | OTC | 12.5 | 0.25 | | |
| 5 | 5 | OTC | 6.25 | 0.125 | | 6 hr post |
| 6 | 5 | OTC | 3.125 | 0.0625 | | 48 hr post |

Twenty four hours following dose administration, groups 1-4 were euthanized. Six hours following dose administration, group 5 was euthanized. Forty eight hours following dose administration, group 6 was euthanized. Following euthanasia, retina were harvested and immunohistochemistry was performed using standard methods. Kinetics and dose response studies were performed.

Figs. 4A, 4B, and 4C illustrate an exemplary detection of expressed OTC protein in the various retinal tissue layers. Fig. 4A shows a schematic diagram of the tissue layers in the retina as they appear from the anterior of the retina to the posterior (lower to upper): the ganglionic cell layer (GCL), the inner plexiform layer (IPL), the inner nuclear layer (INL), the outer plexiform layer (OPL), the outer nuclear layer (ONL), the inner segment photoreceptors (IS), the outer segment photoreceptors (OS), the retinal pigmented epithelium layer (RPE). Fig. 4B shows OTC expression in a retinal cross-section visualized by immunohistochemistry followed by enzymatic detection of target (OTC)-bound antibody. The specific tissue layers are indicated by arrows. OTC can be detected in all the layers of the retina, including portion of the choroid visible at the upper end of the section. Fig. 4C shows no detectable immunostaining in a retinal cross-section of PBS administered rabbit. This data exemplifies the deep tissue penetration and robust expression of mRNA-LNP composition delivered by intravitreal administration. This data therefore demonstrates that the present invention may be used for the treatment of diseases affecting all layers of the retina.

### Example 3. Modeling of an effective dose for mRNA therapy in the eye

This example illustrates that an mRNA dose that is effective in inducing expression of the mRNA-encoded protein throughout the retina in a small mammal such as a mouse can be extrapolated to provide an effective mRNA dose in larger mammals including humans.

The data in Examples 1 and 2 demonstrate that it is possible to successfully extrapolate from an mRNA dose that results in expression of the mRNA-encoded protein throughout the retina of the mouse eye to an mRNA dose that is effective in achieving comparable protein expression in rabbit eyes of much larger size.

Based on the relative anterior-posterior dimensions of human and rabbit eyes (Trivedi RH et al., Investigative Ophthalmology & Visual Science, 43(13) (2002); Silver & Csutak, Investigative Ophthalmology & Visual Science, 51(13) (2010)), it can be deduced that the volume of a human eye is approximately 5 times greater than the volume of the eye of New Zealand white rabbit. A 12.5 µg dose of mRNA resulted in effective expression of the mRNA-encoded protein throughout the various layers of the retina. Given the 5 times greater volume of the human eye, it can be extrapolated that a 62.5 µg dose of mRNA administered to a human eye will similarly result in deep tissue penetration and expression of the mRNA-encoded protein throughout all layers of the retina.

Injection through a narrow gauge needle subjects mRNA to shear stress and can result in fragmentation. The LNPs tested herein can be injected without damaging the mRNA encapsulated within it at concentrations between 0.5 mg/ml to 0.8 mg/ml. Typical volumes administered to human eyes by intravitreal injection range from 30 µl to 100 µl so that doses as high as 80 µg can be administered in a single bolus injection using the LNP formulations tested in examples 1 and 2.

## Claims

1. A composition comprising an effective amount of mRNA encoding a protein for use in the treatment of an ocular disease or disorder in a subject in need thereof, wherein the treatment comprises administering the composition to an eye of a human subject via intravitreal administration or suprachoroidal administration,
wherein the effective amount of mRNA administered to the human subject ranges from 5 µg to 100 µg mRNA,
wherein the mRNA is encapsulated in a lipid nanoparticle, and the lipid nanoparticle comprises one or more cationic lipids, one or more non-cationic lipids and a PEG-modified lipid,
wherein administering the composition results in expression of the protein or the peptide encoded by the mRNA in one or more cells located in the outer nuclear layer (ONL), the inner segment photoreceptors (IS), the outer segment photoreceptors (OS), the retinal pigmented epithelium layer (RPE) of the retinal tissue, the choroid, and/or the sclera of the eye,
wherein the subject is suffering from a disease or disorder affecting the posterior retinal layers or a tissue of the posterior eye, and wherein the disease or disorder is selected from age-related macular degeneration (AMD), cytomegalovirus (CMV) retinitis, Leber's congenital amaurosis, Stargardt disease, Usher disease, chorioretinitis, retinal detachment, uveitis, uvetic macular edema, cyclitis, choroiditis, diffuse uveitis and scleritis.

2. The composition for the use of claim 1, wherein the administering the composition results in expression of the protein or the peptide encoded by the mRNA in the retinal tissue.

3. The composition for the use of claim 1, wherein the administering the composition results in expression of the protein or the peptide encoded by the mRNA in the choroid.

4. The composition for the use of claim 1, wherein the administering the composition results in expression of the protein or the peptide encoded by the mRNA in the sclera.

5. The composition for the use of any one of the preceding claims, wherein the effective amount of mRNA administered to the human subject ranges from 10 µg to 80 µg mRNA.

6. The composition for the use of any one of the preceding claims, wherein the effective amount of mRNA administered to the human subject ranges from 30 µg to 60 µg mRNA.

7. The composition for the use of any one of the preceding claims, wherein the composition is administered to the human subject by intravitreal injection.

8. The composition for the use of any one of the preceding claims, wherein the composition is administered at a volume ranging from 30 µl about to about 100 µl.

9. The composition for the use of any one of the preceding claims, wherein the cationic lipid is selected from a group consisting of cKK-E12, OF-02, C12-200, MC3, DLinDMA, DLinkC2DMA, ICE (Imidazol-based), HGT5000, HGT5001, HGT-5002, HGT4003, DODAC, DDAB, DMRIE, DOSPA, DOGS, DODAP, DODMA and DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, 3-(4-(bis(2-hydroxydodecyl)amino)butyl)-6-(4-((2-hydroxydodecyl)(2-hydroxyundecyl)amino)butyl)-1,4-dioxane-2,5-dione (Target 23), 3-(5-(bis(2-hydroxydodecyl)amino)pentan-2-yl)-6-(5-((2-hydroxydodecyl)(2-hydroxyundecyl)amino)pentan-2-yl)-1,4-dioxane-2,5-dione (Target 24), and combinations thereof.

10. The composition for the use of any one of the preceding claims, wherein the non-cationic lipid is selected from a group consisting of DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine) and DOPG (,2-dioleoyl-sn-glycero-3-phospho-(1'-rac-glycerol)).

11. The composition for the use of any one of the preceding claims, wherein the PEG-modified is lipid selected from derivatized ceramides such as N-Octanoyl-Sphingosine-l-[Succinyl(Methoxy Polyethylene Glycol)-2000] (C8 PEG-2000 ceramide); PEG-modified lipids having a polyethylene glycol chain of up to 5 kDa in length covalently attached to a lipid with alkyl chain(s) of C₆-C₂₀ length, a PEGylated cholesterol and PEG-2K.

12. The composition for the use of any one of the preceding claims, wherein the lipid component of the lipid nanoparticle consists of a cationic lipid, a non-cationic lipid, cholesterol and a PEG-modified lipid.

13. The composition for the use of any one of the preceding claims, wherein:
(a) the cationic lipid constitutes about 30-70 % of the lipid nanoparticle by molar ratio, and/or
(b) the PEG-modified lipid comprises at least 1%, at least 2%, at least 3%, at least 4%, at least 5%, at least 6%, at least 7%, at least 8%, at least 9%, or at least 10% of the total lipids in the lipid nanoparticle.

14. The composition for the use of any one of the preceding claims, wherein the ratio of cationic lipid(s) to non-cationic lipid(s) to cholesterol-based lipid(s) to PEG-modified lipid(s) is between about 30-60:25-35:20-30:1-15.

15. The composition for the use of any one of the preceding claims, wherein:
(a) the mRNA encodes a protein or a peptide selected from a group consisting of an ocular protein or a peptide, a vaccine, an antibody or a fragment thereof, a hormone, a structural protein or peptide, an extracellular matrix protein or peptide, a vascular protein or peptide, an antioxidant protein or peptide, a receptor protein or peptide, a signaling protein or peptide, a transcription factor and an enzyme
(b) the mRNA encodes an ocular protein or a peptide selected from a group consisting of ADAM metallopeptidase domain 9, adhesins, ATP synthase, bestrophin 1, cadherins, chemokines, ciliary neurotrophic factor, collagens, complement factors, cytochromes, IGF, metalloproteinases, mitofusin, NADH dehydrogenase, OPA1, PDGF, peripherin 2, retinoschisin, SOD2, thrombospondin receptor, and vascular endothelial growth factor (VEGF), and/or
(c) the mRNA encodes an antibody or a fragment thereof, that binds to ADAM metallopeptidase domain 9, adhesins, ATP synthase, bestrophin 1, cadherins, chemokines, ciliary neurotrophic factor, collagens, complement factors, cytochromes, IGF, metalloproteinases, mitofusin, NADH dehydrogenase, OPA1, PDGF, peripherin 2, retinoschisin, SOD2, thrombospondin receptor, or vascular endothelial growth factor (VEGF), optionally wherein the mRNA encodes an antibody or a fragment thereof that binds to VEGF.

## Patentansprüche

1. Zusammensetzung, umfassend eine wirksame Menge an ein Protein codierender mRNA, zur Verwendung bei der Behandlung einer Augenerkrankung oder -störung bei einem behandlungsbedürftigen Individuum, wobei die Behandlung Verabreichen der Zusammensetzung an ein Auge eines menschlichen Individuums über intravitreale Verabreichung oder suprachoroidale Verabreichung umfasst,
wobei die wirksame Menge an mRNA, die dem menschlichen Individuum verabreicht wird, im Bereich von 5 µg bis 100 µg mRNA liegt,
wobei die mRNA in einem Lipidnanopartikel verkapselt ist und das Lipidnanopartikel ein oder mehrere kationische Lipide, ein oder mehrere nicht-kationische Lipide und ein PEG-modifiziertes Lipid umfasst,
wobei das Verabreichen der Zusammensetzung zur Expression des durch die mRNA codierten Proteins oder Peptids in einer oder mehreren Zellen in der äußeren Körnerschicht (Outer Nuclear Layer, ONL), den Innensegment-Photorezeptoren (IS), den Außensegment-Photorezeptoren (OS), dem retinalen Pigmentepithel (RPE) des Netzhautgewebes, der Aderhaut und/oder der Lederhaut des Auges führt,
wobei das Individuum an einer Erkrankung oder Störung leidet, die die hinteren Netzhautschichten oder ein Gewebe des hinteren Auges betrifft, und wobei die Erkrankung oder Störung aus altersbedingter Makuladegeneration (AMD), Cytomegalievirus(CMV)-Retinitis, Leberscher kongenitaler Amaurose, Morbus Stargardt, Usher-Syndrom, Chorioretinitis, Netzhautablösung, Uveitis, uveitischem Makulaödem, Zyklitis, Choroiditis, diffuser Uveitis und Skleritis ausgewählt ist.

2. Zusammensetzung zur Verwendung nach Anspruch 1, wobei das Verabreichen der Zusammensetzung zur Expression des durch die mRNA codierten Proteins oder Peptids im Netzhautgewebe führt.

3. Zusammensetzung zur Verwendung nach Anspruch 1, wobei das Verabreichen der Zusammensetzung zur Expression des durch die mRNA codierten Proteins oder Peptids in der Aderhaut führt.

4. Zusammensetzung zur Verwendung nach Anspruch 1, wobei das Verabreichen der Zusammensetzung zur Expression des durch die mRNA codierten Proteins oder Peptids in der Lederhaut führt.

5. Zusammensetzung zur Verwendung nach einem der vorhergehenden Ansprüche, wobei die wirksame Menge an mRNA, die dem menschlichen Individuum verabreicht wird, im Bereich von 10 µg bis 80 µg mRNA liegt.

6. Zusammensetzung zur Verwendung nach einem der vorhergehenden Ansprüche, wobei die wirksame Menge an mRNA, die dem menschlichen Individuum verabreicht wird, im Bereich von 30 µg bis 60 µg mRNA liegt.

7. Zusammensetzung zur Verwendung nach einem der vorhergehenden Ansprüche, wobei die Zusammensetzung dem menschlichen Individuum durch intravitreale Injektion verabreicht wird.

8. Zusammensetzung zur Verwendung nach einem der vorhergehenden Ansprüche, wobei die Zusammensetzung in einem Volumen im Bereich von 30 µl etwa bis etwa 100 µl verabreicht wird.

9. Zusammensetzung zur Verwendung nach einem der vorhergehenden Ansprüche, wobei das kationische Lipid ausgewählt ist aus einer Gruppe bestehend aus cKK-E12, OF-02, C12-200, MC3, DLinDMA, DLinkC2DMA, ICE (Imidazolbasiert), HGT5000, HGT5001, HGT-5002, HGT4003, DODAC, DDAB, DMRIE, DOSPA, DOGS, DODAP, DODMA und DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, 3-(4-(Bis(2-hydroxydodecyl)amino)butyl)-6-(4-((2-hydroxydodecyl)(2-hydroxyundecyl)amino)butyl)-1,4-dioxan-2,5-dion (Ziel 23), 3-(5-(Bis(2-hydroxydodecyl)amino)pentan-2-yl)-6-(5-((2-hydroxydodecyl)(2-hydroxyundecyl)amino)pentan-2-yl)-1,4-dioxan-2,5-dion (Ziel 24) und Kombinationen davon.

10. Zusammensetzung zur Verwendung nach einem der vorhergehenden Ansprüche, wobei das nicht-kationische Lipid ausgewählt ist aus einer Gruppe bestehend aus DSPC (1,2-Distearoyl-sn-glycero-3-phosphocholin), DPPC (1,2-Dipalmitoyl-sn-glycero-3-phosphocholin), DOPE (1,2-Dioleyl-sn-glycero-3-phosphoethanolamin), DOPC (1,2-Dioleyl-sn-glycero-3-phosphotidylcholin) DPPE (1,2-Dipalmitoyl-sn-glycero-3-phosphoethanolamin), DMPE (1,2-Dimyristoyl-sn-glycero-3-phosphoethanolamin) und DOPG (,2-Dioleoyl-*sn*-glycero-3-phospho-(1'-*rac*-glycerin)).

11. Zusammensetzung zur Verwendung nach einem der vorhergehenden Ansprüche, wobei es sich bei dem PEG-modifizierten um ein Lipid handelt, das ausgewählt ist aus derivatisierten Ceramiden wie N-Octanoyl-Sphingosine-1-[Succinyl(Methoxy Polyethylene Glycol)-2000] (C8-PEG-2000-Ceramid), PEG-modifizierten Lipiden mit einer Polyethylenglykolkette mit einer Länge von bis zu 5 kDa ist, die kovalent an ein Lipid mit einer oder mehreren Alkylketten der Länge C₆-C₂₀ gebunden ist, einem PEGylierten Cholesterin und PEG-2K.

12. Zusammensetzung zur Verwendung nach einem der vorhergehenden Ansprüche, wobei die Lipidkomponente des Lipidnanopartikels aus einem kationischen Lipid, einem nicht-kationischen Lipid, Cholesterin und einem PEG-modifizierten Lipid besteht.

13. Zusammensetzung zur Verwendung nach einem der vorstehenden Ansprüche, wobei:
(a) das kationische Lipid etwa 30-70 % des Lipidnanopartikels nach Molverhältnis ausmacht und/oder
(b) das PEG-modifizierte Lipid mindestens 1 %, mindestens 2 %, mindestens 3 %, mindestens 4 %, mindestens 5 %, mindestens 6 %, mindestens 7 %, mindestens 8 %, mindestens 9 % oder mindestens 10 % der gesamten Lipide im Lipidnanopartikel umfasst.

14. Zusammensetzung zur Verwendung nach einem der vorhergehenden Ansprüche, wobei das Verhältnis von kationischem Lipid (kationischen Lipiden) zu nichtkationischem Lipid (nicht-kationischen Lipiden) zu Lipid(en) auf Cholesterinbasis zu PEG-modifiziertem Lipid (PEG-modifizierten Lipiden) zwischen etwa 30-60:25-35:20-30:1-15 liegt.

15. Zusammensetzung zur Verwendung nach einem der vorstehenden Ansprüche, wobei:
(a) die mRNA ein Protein oder ein Peptid codiert, das ausgewählt ist aus einer Gruppe bestehend aus einem Augenprotein oder einem Peptid, einem Impfstoff, einem Antikörper oder einem Fragment davon, einem Hormon, einem Strukturprotein oder -peptid, einem extrazellulären Matrixprotein oder -peptid, einem Gefäßprotein oder - peptid, einem Antioxidans-Protein oder -Peptid, einem Rezeptorprotein oder -peptid, einem Signalgebungsprotein oder -peptid, einem Transkriptionsfaktor und einem Enzym,
(b) die mRNA ein Augenprotein oder ein Peptid codiert, das ausgewählt ist aus einer Gruppe bestehend aus ADAM-Metallopeptidase-Domäne 9, Adhäsinen, ATP-Synthase, Bestrophin 1, Cadherinen, Chemokinen, CNTF (Ciliary Neurotrophic Factor), Kollagenen, Komplementfaktoren, Cytochromen, IGF, Metalloproteinasen, Mitofusin, NADH-Dehydrogenase, OPA1, PDGF, Peripherin 2, Retinoschisin, SOD2, Thrombospondin-Rezeptor und VEGF (Vascular Endothelial Growth Factor), und/oder
(c) die mRNA einen Antikörper oder ein Fragment davon codiert, der/das an die ADAM-Metallopeptidase-Domäne 9, Adhäsine, ATP-Synthase, Bestrophin 1, Cadherine, Chemokine, CNTF (Ciliary Neurotrophic Factor), Kollagene, Komplementfaktoren, Cytochrome, IGF, Metalloproteinasen, Mitofusin, NADH-Dehydrogenase, OPA1, PDGF, Peripherin 2, Retinoschisin, SOD2, Thrombospondin-Rezeptor oder VEGF (Vascular Endothelial Growth Factor) bindet, gegebenenfalls wobei die mRNA einen Antikörper oder ein Fragment davon codiert, der/das an VEGF bindet.

## Revendications

1. Composition comprenant une quantité efficace d'ARNm codant pour une protéine destinée à être utilisée dans le traitement d'une maladie ou d'un trouble oculaire chez un sujet qui en a besoin, dans laquelle le traitement comprend l'administration de la composition à un œil d'un sujet humain par administration intravitréenne ou par administration suprachoroïdienne,
dans laquelle la quantité efficace d'ARNm administrée au sujet humain est dans la plage de 5 µg à 100 µg d'ARNm, dans laquelle l'ARNm est encapsulé dans une nanoparticule lipidique, et la nanoparticule lipidique comprend un ou plusieurs lipides cationiques, un ou plusieurs lipides non cationiques et un lipide modifié par PEG,
dans laquelle l'administration de la composition résulte en l'expression de la protéine ou du peptide codé(e) par l'ARNm dans une ou plusieurs cellules situées dans la couche nucléaire externe (ONL), les photorécepteurs du segment interne (IS), les photorécepteurs du segment externe (OS), la couche épithéliale pigmentée rétinienne (RPE) du tissu rétinien, la choroïde, et/ou la sclère de l'œil,
dans laquelle le sujet souffre d'une maladie ou un trouble affectant les couches rétiniennes postérieures ou un tissu de l'œil postérieur, et dans laquelle la maladie ou le trouble est choisi(e) parmi la dégénérescence maculaire liée à l'âge (DMLA), la rétinite à cytomégalovirus (CMV), l'amaurose congénitale de Leber, la maladie de Stargardt, la maladie d'Usher, la choriorétinite, le décollement de la rétine, l'uvéite, l'œdème maculaire uvéitique, la cyclite, la choroïdite, l'uvéite diffuse et la sclérite.

2. Composition pour l'utilisation selon la revendication 1, dans laquelle l'administration de la composition a pour résultat l'expression de la protéine ou du peptide codé(e) par l'ARNm dans le tissu rétinien.

3. Composition pour l'utilisation selon la revendication 1, dans laquelle l'administration de la composition a pour résultat l'expression de la protéine ou du peptide codé(e) par l'ARNm dans la choroïde.

4. Composition pour l'utilisation selon la revendication 1, dans laquelle l'administration de la composition a pour résultat l'expression de la protéine ou du peptide codé(e) par l'ARNm dans la sclère.

5. Composition pour l'utilisation de l'une quelconque des revendications précédentes, dans laquelle la quantité efficace d'ARNm administrée au sujet humain est dans la plage de 10 µg à 80 µg d'ARNm.

6. Composition pour l'utilisation de l'une quelconque des revendications précédentes, dans laquelle la quantité efficace d'ARNm administrée au sujet humain est dans la plage de 30 µg à 60 µg d'ARNm.

7. Composition pour l'utilisation de l'une quelconque des revendications précédentes, dans laquelle la composition est administrée au sujet humain par injection intravitréenne.

8. Composition pour l'utilisation selon l'une quelconque des revendications précédentes, dans laquelle la composition est administrée à un volume allant de 30 µl environ à environ 100 µl.

9. Composition pour l'utilisation selon l'une quelconque des revendications précédentes, dans laquelle le lipide cationique est choisi dans un groupe constitué par cKK-E12, OF-02, C12-200, MC3, DLinDMA, DLinkC2DMA, ICE (à base d'imidazole), HGT5000, HGT5001, HGT-5002, HGT4003, DODAC, DDAB, DMRIE, DOSPA, DOGS, DODAP, DODMA et DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, 3-(4-(bis(2-hydroxydodécyl)amino)butyl)-6-(4-((2-hydroxydodécyl)(2-hydroxyundécyl)amino)butyl)-1,4-dioxane-2,5-dione (cible 23), 3-(5-(bis(2-hydroxydodécyl)amino)pentan-2-yl)-6-(5-((2-hydroxydodécyl)(2-hydroxyundécyl)amino)pentan-2-yl)-1,4-dioxane-2,5-dione (cible 24), et leurs combinaisons.

10. Composition pour l'utilisation selon l'une quelconque des revendications précédentes, dans laquelle le lipide non cationique est choisi dans un groupe constitué par DSPC (1,2-distéaroyl-sn-glycéro-3-phosphocholine), DPPC (1,2-dipalmitoyl-sn-glycéro-3-phosphocholine), DOPE (1,2-dioléoyl-sn-glycéro-3-phosphoéthanolamine), DOPC (1,2-dioléoyl-sn-glycéro-3-phosphotidylcholine), DPPE (1,2-dipalmitoyl-sn-glycéro-3-phosphoéthanolamine), DMPE (1,2-dimyristoyl-sn-glycéro-3-phosphoéthanolamine) et DOPG (1,2-dioléoyl-sn-glycéro-3-phospho-(1'-rac-glycérol)).

11. Composition pour l'utilisation selon l'une quelconque des revendications précédentes, dans laquelle le lipide modifié par PEG est choisi parmi les céramides dérivatisés tels que N-octanoyl-sphingosine-1-[succinyl(méthoxy-polyéthylène glycol)-2000] (C8 PEG-2000 céramide) ; les lipides modifiés par PEG ayant une chaîne polyéthylène glycol d'une longueur allant jusqu'à 5 kDa fixée de manière covalente à un lipide par une ou plusieurs chaînes alkyle d'une longueur de C₆-C₂₀, un cholestérol PEGylé et PEG-2K.

12. Composition pour l'utilisation selon l'une quelconque des revendications précédentes, dans laquelle le composant lipidique de la nanoparticule lipidique est constitué d'un lipide cationique, d'un lipide non cationique, de cholestérol et d'un lipide modifié par PEG.

13. Composition pour l'utilisation selon l'une quelconque des revendications précédentes, dans laquelle :
(a) le lipide cationique constitue environ 30-70 % de la nanoparticule lipidique en rapport molaire, et/ou
(b) le lipide modifié par PEG comprend au moins 1 %, au moins 2 %, au moins 3 %, au moins 4 %, au moins 5 %, au moins 6 %, au moins 7 %, au moins 8 %, au moins 9 %, ou au moins 10 % des lipides totaux dans la nanoparticule lipidique.

14. Composition pour l'utilisation selon l'une quelconque des revendications précédentes, dans laquelle le rapport du ou des lipide(s) cationique(s) sur le ou les lipide(s) non cationique(s) sur le ou les lipide(s) à base de cholestérol sur le ou les lipide(s) modifié(s) par PEG est compris entre environ 30-60:25-35:20-30:1-15.

15. Composition pour l'utilisation selon l'une quelconque des revendications précédentes, dans laquelle :
(a) l'ARNm code pour une protéine ou un peptide choisi(e) dans un groupe constitué par une protéine oculaire ou un peptide, un vaccin, un anticorps ou un fragment de celui-ci, une hormone, une protéine ou un peptide structural(e), une protéine ou un peptide de matrice extracellulaire, une protéine ou un peptide vasculaire, une protéine ou un peptide antioxydant(e), une protéine ou un peptide réceptrice/récepteur, une protéine ou un peptide de signalisation, un facteur de transcription et une enzyme
(b) l'ARNm code pour une protéine oculaire ou un peptide choisi(e) dans un groupe constitué le domaine 9 de la métallopeptidase ADAM, les adhésines, l'ATP synthase, la bestrophine 1, les cadhérines, les chimiokines, le facteur neurotrophique ciliaire, les collagènes, les facteurs du complément, les cytochromes, IGF, les métalloprotéinases, la mitofusine, la NADH déshydrogénase, OPA1, PDGF, la périphérine 2, la rétinoscisine, SOD2, le récepteur de la thrombospondine et le facteur de croissance endothélial vasculaire (VEGF), et/ou
(c) l'ARNm code pour un anticorps ou un fragment de celui-ci, qui se lie au domaine 9 de la métallopeptidase ADAM, aux adhésines, à l'ATP synthase, à la bestrophine 1, aux cadhérines, aux chimiokines, au facteur neurotrophique ciliaire, aux collagènes, aux facteurs du complément, aux cytochromes, à l'IGF, aux métalloprotéinases, à la mitofusine, à la NADH déshydrogénase, à l'OPA1, à PDGF, à la périphérine 2, à la rétinoschisine, à SOD2, au récepteur de la thrombospondine ou au facteur de croissance endothélial vasculaire (VEGF), éventuellement dans laquelle l'ARNm code pour un anticorps ou un fragment de celui-ci qui se lie à VEGF.
